# EUROPEAN PATENT SPECIFICATION

(11) **EP 0 439 585 B1**
(45) Date of publication and mention of the grant of the patent: **29.10.1997**
(21) Application number: 90912436.4
(22) Date of filing: 18.08.1990
(51) Int. Cl.: C08J 7/12, C08J 7/00, C07K 17/06, C12N 11/06, G01N 33/543, C12Q 1/68

(54) **IMMOBILISATION OF LIGANDS BY RADIO-DERIVATIZED POLYMERS**
IMMOBILISIERUNG VON MOLEKÜLEN DURCH VERWENDUNG VON RADIODERIVATISIERTEN POLYMEREN
IMMOBILISATION DE LIGANDS A L'AIDE DE POLYMERES RADIO-TRANSFORMES EN DERIVES

(30) Priority: 21.08.1989 AT 1976/89; 09.04.1990 US 507348
(43) Date of publication of application: 07.08.1991
(73) Proprietor: Mag.Georg Mayrhofer & Co.OHG, 4020 Linz (AT)
(72) Inventor: VARGA, Janos, M., A-6020 Innsbruck (AT); FRITSCH, Peter, A-6020 Innsbruck (AT)
(74) Representative: Itze, Peter, Dipl.-Ing.
(86) International application number: EP9001362
(87) International publication number: WO9102768

## Description

### BACKGROUND OF THE INVENTION

There is a need for covalent modification of polymer surfaces to be used as carriers of molecules, as support for cells or as sorbents in separation methods. The limitations of adsorptive coating of polystyrene, herein referred to as PS, which can be used, for example, as microtiter plates or for bio-assays, are generally known and the need for covalent coupling methods is recognized in the art.

Functionalized PS suitable for covalent attachment of ligands and antibodies has been obtained by the following three methods: copolymerization of monomers containing functional groups as disclosed in Lloyd and Durscher, J. Appl. Polymer. Sci., Vol. 7, 1963, pp. 2025-2037; chemical modification of PS products as taught by Chu and Tarcha, P.J., J. Appl. Polymer. Sci., Vol. 34, 1987, pp. 1912-1924, and graft-polymerization of unsaturated monomers as demonstrated in EP 0 106 169. One problem with these procedures is that the resulting products have impaired optical qualities and/or high background noise in binding assays.

Only the last method involving graft-polymerization of unsaturated monomers uses radiation to initiate covalent bond formation between the support material and the spacer or molecules to be immobilized. Radiation graft-polmerization uses polymerizable monomers to build up a chain in a radiation-initiated or free radical polymerization reaction. The disadvantages associated with radiation graft-polymerization are as follows: 1) Graft-polymerization is technically difficult to do and it is not easy to control, i.e., the length of the grafted polymer is variable; 2) During the process of graft-polymerization other reactions, such as cross-linking of the grafted polymer chain may take place in addition to polymerization resulting in a rigid radiation-grafted layer of polymer; 3) In cases when the ligating group is attached only to the end of the grafted chain, graft-polymerization is not expected to yield high capacity of support; 4) Since in ligand-antibody interactions approximately 10 Å length spacers have been found optimal, See Lowe et al., Biochem. J., Vol.133, 1973, pp. 499-514, the long (hundreds to thousands of monomer length) grafted chains obtained in EP 0 106 769 are suboptimal; 5) In most graft-polymerization methods large areas of charged and/or hydrophobic surfaces are introduced, leading to high levels of nonspecific interactions and unacceptable background noise, See, Lowe and Dean, P.D.G. Affinity Chromatography, J.Wiley and Sons, N.Y. 1974. These disadvantages of radiation graft-polymerization can be circumvented by using nonpolymerizable molecules for radiation-mediated derivatization of polymers as described below.

Akerman et al (WO 87/02619) discloses assay supports that are treated with abrasion, laser light or high voltage to improve the polymer support's capacity for binding ligands. No conjugands are used in the methods disclosed. Glutaraldehyde is disclosed in Akerman et al on pages 7 and 8 as a bifunctional coupling agent for enhancing the binding capacity of the support and not as a conjugand.

Also, the three treatments disclosed in Akerman et al -- abrasion, laser light and high voltage -- do not involve high energy radiation and operate by mechanisms that differ from the mechanism involved in applicants' high energy radio-derivatization. Abrasion is a mechaical process that enhances binding capacity by increasing the effective surface area of the polymeric support. Thus, the polymeric surface is physically changed and not chemically modified or derivatized as in the present invention.

Krämer et al (DE-AS 22 63 289) teaches the covalent attachment of polypeptides to various carriers in the absence of oxygen and in the presence of photosensitizers and UV light, or in the presence of organic peroxides, photosensitizers and visible light. The essence of this method is photochemical immobilization of molecules.

The photochemical activation method in Krämer et al operates by a different mechanism for the high energy-radio-derivatization method of applicants' invention. Photochemical activation is achieved by using electromagnetic radiation in the ultra-violet region, i.e., having a wavelength of 10-400 nm. This radiation has a relatively low level of energy (approximately 5-100 eV) as compared to the high energy (for example, gamma radiation, which has an energy level of at least 100,000eV).

### Summary of the Invention

The invention relates to a method of using nonpolymerizable small molecules in the radiation-mediated derivatization (radio-derivatization) of polymers. When polymers such as molded PS products (e.g. microtiter plates) or latex particles are irradiated with high energy, selected from gamma rays with an irradiation dose in the range of 1 to 15 Mrads and an irradiation intensity of 0,1 to 0,2 Mrads/h and electron-radiation that is capable of radiolysing the polymer and/or conjugand in the presence of nonpolymerizable small molecules, such as aromatic amines, some of these molecules incorporate into the polymer leading to the formation of radio-derivatized polymer, which is herein referred to as RDP. Having improved adsorptive and ion-binding characteristics, RDPs can be used directly as cell/tissue culture substrates, ion-exchangers, or sorbents for chromatography.

This invention further relates to RDPs suitable for introducing anchoring groups to be used for covalent immobilization or fixing of molecules on polymer surfaces. Two classes of RDPs can be identified regarding their ability for covalent immobilization of biologically important molecules: 1) reactive RDPs, which are able to form covalent bonds with molecules such as proteins without the help of cross-linkers and 2) functionalized RDPs, which can be used for the immobilization of molecules with activators (e.g. carbodiimides) or with cross-linkers.

It is an object of the present invention to produce solid, low-noise supports having favorable adsorptive characteristics for binding assays. Most of the RDPs can be produced without the impairment of the optical quality of transparent polymers, e.g. PS, thereby making derivatized microtiter plates suitable for colorimetric assays. RDPs in particle form are suitable for the preparation of affinity sorbents, e.g., for high performance affinity chromatography and for the immobilization of enzymes.

The problem solved with radio-derivatization is: derivatization of polymers with the help of high-energy radiation. The purpose of this procedure is 1) to provide anchoring groups on the surface of polymers as in functionalized polymers, that can be used for the covalent attachment of ligates or ligands (such as proteins, DNA, etc) in the presence of cross-linkers or activators, or 2) to produce reactive polymers that can be used for the covalent attachment of ligates and ligands in one step without cross-linkers and activators.

### Brief Description of the Drawings

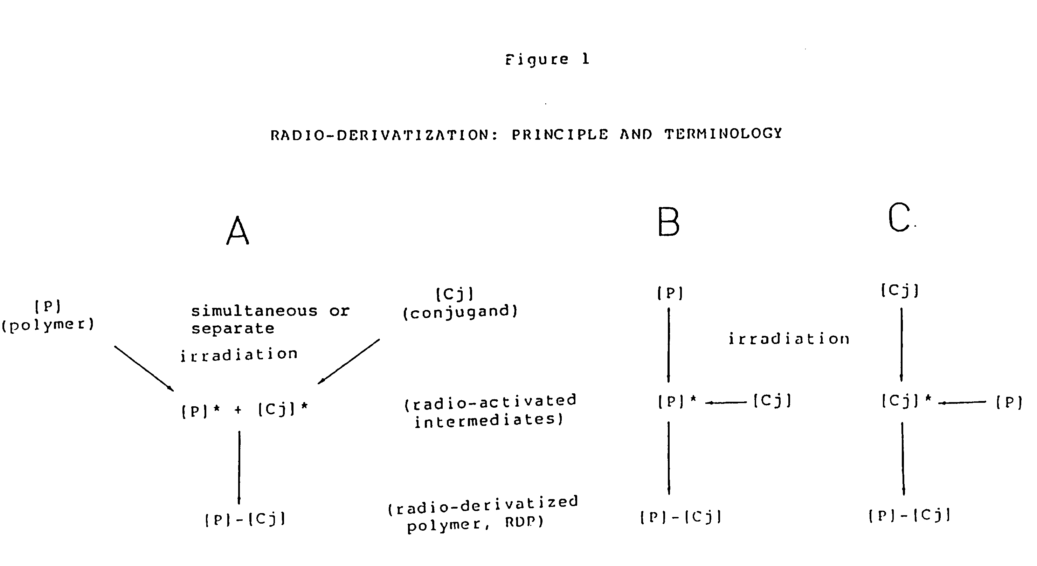

Figure 1 shows the basic principles of radio-derivatization according to this invention and the four ways of obtaining radio-derivatization polymers.

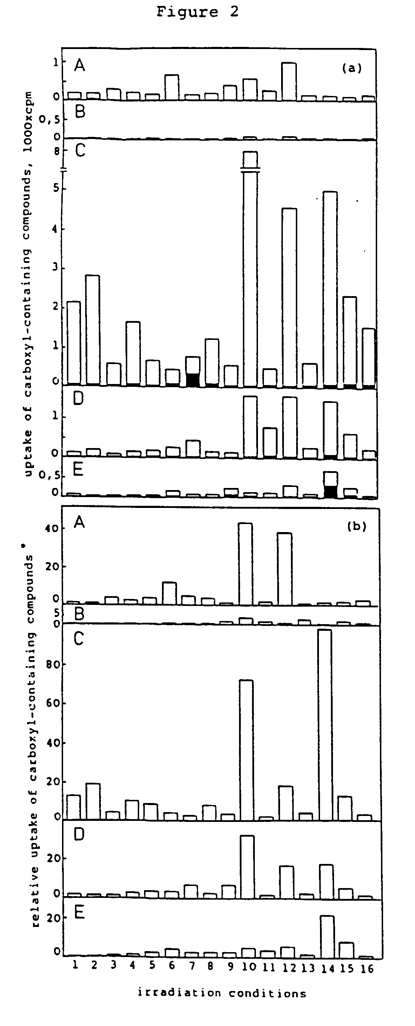

Figure 2 shows the effects of radio-derivatization of polystyrene on the EDC-mediated uptake of five carboxylic acids designated A through E. In the top chart designated (a), shaded histograms show uptake of radio activity without ethyldiethylaminopropyl-carbodiimide (EDC). The lower chart (b) illustrates the relative uptake of radioactivity, which is the ratio of uptake obtained with irradiated plates versus non-irradiated plates in the presence of EDC. Figure 2 is explained in more detail in Example 4.

### DETAILED DESCRIPTION OF THE PREFERRED EMBODIMENT

The principles of the present invention are illustrated in Figure 1. The term radio-derivatization describes the radiation-mediated stable attachment of nonpolymerizable molecules to polymers. Within this term, "radio-" stands for irradiation in the same way as radiotherapy. In schemes A, B, and C of Figure 1, [P] can be any polymer that contains radiolysable bonds. Conjugands, designated as [Cj], can be any compound containing radiolysable bonds. In particular, aromatic compounds are preferable with polystyrene. Radio activated intermediates [P]* and [Cj]* can be free radicals and/or other reactive intermediates of [P] and [Cj] that in recombinations, condensations or other types of reactions lead to the formation of radio-derivatized products.

The radio-derivatized polymer (RDP) is represented as [P]-[Cj], which is a collective term to denote the union of [P] and [Cj] supposedly by covalent bond formation. Even if non-covalent interactions--such as entrapment of Cj in P--contribute to the formation of some of the radio-derivatives, these have been shown to be stable enough for the immobilization of molecules for being practically useful in solid-phase assays (Example 22). Reactive RDPs are those RDPs that are able to form stable bond(s) with molecules of practical importance such as ligands, antibodies and enzymes in the absence of activators or cross-linkers. Functionalized RDPs are RDPs carrying functional groups such as nucleophiles. Additional substances such as activators or cross-linkers are needed for the covalent attachment of molecules of practical importance to functionalized RDP.

Radio-derivatization can be carried out in four different methods, each of which is believed to result in the formation of the same RPD. Two methods are depicted in Figure 1A, firstly, simultaneous radio-derivatization wherein [P] and [Cj] are irradiated in close contact with each other (i.e., microtiter plate wells containing a solution of Cj) and, secondly, separate radio-derivatization wherein [P] and [Cj] are irradiated separately to form [P]* and [Cj]*, which are then brought into contact. A third approach is shown in Figure 1B wherein only [P] is irradiated to form [P]* which is then exposed to [Cj]. Figure 1C shows a fourth way wherein only [Cj] is irradiated to form [Cj]*, which is then exposed to [P].

While the examples described herein were all performed using the simultaneous method (Figure 1 A), any of the other three approaches can be used. The formation of stable, long-lasting radicals and/or other reactive intermediates during high energy irradiation is well known (hexadienylradicals, formed from polystyrene by high energy gamma radiation may survive for months, i.e., the half-life of these radicals is approximately one-half year. See Wilske and Heusinger, J. Polymer. Sci., Vol. 7, 1969, pp. 995-1010). The sequential process(es) may be preferred when the structural integrity of [Cj] or [P] is to be preserved.

The polymers of this invention are those polymers containing radiolysable bonds, which undergo radiolysis under the conditions of radio-derivatization. In the context of this invention, radiolysable means sufficient susceptibility to radiolysis to provide activated foci on the surface of a polymer in densities to allow coating of the plate with approximately 100 p. moles/cm² anchoring functions when irradiated at doses of 1-10 Mrads. Although the preferred polymer in this invention is polystyrene and its co-polymers, other suitable radiolysable polymers include acetal resins, acrylics and their co-polymers, alkyl resins, cellulose acetate, coumarone-indene resins, epoxy resins, furane resins, melamine resins, paracoumarone-indene resins, pentaerythritol resins, phenolics and their copolymers, polyesters and their co-polymers, polyethylene, and its copolymers, polypropylene and its co-polymers, polyamides (nylon, etc.), polybutadienes and their copolymers (butadiene-styrene), polyethylene glycols, polyvinyl alcohol and its co-polymers, polyvinyl chloride and its co-polymers, polyvinyl acetate and its co-polymers, polyphtalates and their co-polymers, polyurethane and its co-polymers, polyisoprene and its co-polymers, silicones and urea-formaldehyde polymers.

The term "conjugand" as used herein, is a collective term that refers to the nonpolymerizable compound that is initially linked to the polymer to form the RDP in radio-derivatization. These nonpolymerizable compounds are chemically pure and contain radiolysable bonds. The term radiolysable is defined above and the term nonpolymerizable as used herein means a compound that does not undergo polymerization--or if it does, polymerization occurs as an unintended side reaction--during exposure to high-energy irradiation.

Depending on the type of conjugand used, the present invention produces two classes of RDPs: reactive RDPs and functionalized RDPs. Reactive RDPs can be used for the immobilization of molecules such as proteins without activators or cross-linkers. They can be produced by generation of reactive groups on the polymers from nonreactive components during the process of radio-derivatization as in Example 3, Table 3. The exact chemical nature and mechanisms for the formation of these products is unknown. However, without being bound by theory, it is believed that during irradiation of polystyrene in the presence of water, oxygen and aromatic compounds, such as p-aminophenol or 2-amino-4-Cl-phenol, protein-reactive groups, such as aldehydes and/or quinones are generated. Reactive RDPs can also be obtained by radio-derivitization with radiation-resistant acid-halides, acid-azides or active esters that are able to react with suitable functions such as nucleophilic groups present on proteins and other biologically important molecules. Reactive RDPs can also be produced by the activation of functionalized RDPs.

Reactive RDPs can be prepared by irradiating polymers in the presence of nonpolymerizable conjugands that are quinones or compounds from which quinones or quinoid structures are generated during radio-derivatization. General examples of such conjugands are aromatic polyols, amino-phenols and certain aromatic diamines. Particularly preferred conjugands in this class are p-aminophenol, 2-amino-4-Cl-phenol and 5-I-2-amino-pyrimidin. Specific examples of reactive RDP forming conjugands which are quinones or compounds from which quinones are generated are: acenaphtene quinone, acetyl pyrocatechol, 4-acetyl pyrogallol, alizarin, 3-amino alizarin, 4-amino alizarin, 2,3-dihydroxy aniline, 3,4-dihydroxy aniline, 1,2-dihydroxy anthracene, 9,10-dihydroxy anthracene, 9,10-anthraquinone, 1-amino-9,10-anthraquinone, 2-amino-9,10-anthraquinone, 1-bromo-9,10-anthraquinone, 2-bromo-9,10-anthraquinone, 1-chloro-9,10-anthraquinone, 2-chloro-9,10-anthraquinone, 1-hydroxy anthraquinone, 2-hydroxy anthraquinone, 1,4-dihydroxy anthraquinone, 4,5-dihydroxy anthraquinone, 3,4-dihydroxy benzaldehyde, 1-chloro 2,3-dihydroxy benzene, 4-chloro 1,2-dihydroxy benzene, 1,2-diamino benzene, 1,4-diamino benzene, o-aminophenol, p-aminophenol, 3,4-diamino benzene sulfuric acid, 2,3-diamino benzoic acid, 2,3-dihydroxy benzoic acid, 3,4-diamino biphenyl, 3,4-dihydroxy benzoic acid, 2,3-dihydroxy benzophenone, chloranil, chloranilic acid, catechol, homogentistic acid, hydroquinone, 2-acetyl hydroquinone, isatin, 9,10-naphthacene quinone, 9,11-naphthacene quinone, 1,4-diamino naphthalene, 1,2-diamino naphthalene, 2,3-diamino naphthalene, 1,4-diamino-2-methyl naphthalene, 1,2-dihydroxy naphthalene, 1,4-dihydroxy naphthalene, 2,3-dihydroxy naphthalene, 1,2-naphthoquinone, 3-bromo 1,2-naphthoquinone, 4-bromo 1,2-naphthoquinone, 6-bromo 1,2-naphthoquinone, 3-chloro 1,2-naphthoquinone, 3,6-dibromo 1,2-naphthoquinone, 4,6-dibromo 1,2-naphthoquinone, 6-hydroxy 1,2 naphthoquinone, 7-hydroxy 1,2-naphthoquinone, 3-methyl 1,2 naphthoquinone, 4-methyl 1,2-naphthoquinone, 1,4-naphthoquinone, 2-chloro 1,4-naphthoquinone, 5-chloro 1,4-naphthoquinone, 6-chloro 1,4-naphthoquinone, 2,6-dichloro 1,4-naphthoquinone, 5,6-dichloro 1,4-naphthoquinone, 5,8-dichloro 1,4-naphthoquinone, 5,8-dihydroxy 1,4-naphthoquinone, 2,5-dimethyl 1,4-naphthoquinone, 2,6-dimethyl 1,4-naphthoquinone, 2,8-dimethyl 1,4-naphthoquinone, 2-ethyl-1,4-naphthoquinone, 2-hydroxy 1,4-naphthoquinone, 5-hydroxy 1,4-naphthoquinone, 6-hydroxy 1,4-naphthoquinone, menadione, 2-phenyl 1,4-naphthoquinone, nitranilic acid, naphthopurpurin, 3,4-dihydroxy phenanthrene, 9,10-phenanthraquinone, 1,2-dihydroxy 9,10-phenanthraquinone, 2-hydroxy 9,10-phenanthraquinone, 4,5-dihydroxy, 9,10-phenanthraquinone, 3-hydroxy, 9,10-phenanthraquinone, o-quinone, 3-chloro o-quinone, 4-chloro o-quinone, p-quinone, 2-chloro p-quinone, toluquinone, phenyl p-quinone, 2,3-benzothio phenequinone, thiosatin, 2,3-dihydroxy toluene, and 3,4-dihydroxy toluene.

In addition to the above, conjugands that are protein modifying reagents can be used to prepare reactive RDPs.

Generally, protein modifying reagents that are known in the art can serve as conjugands for preparing reactive RDPs. Specific examples of suitable protein modifying reagents are: acetic anhydride, acetaldehyde, acetaldehyde bis(2-chloroethyl)acetal, acetaldehyde diacetal, acetaldehyde dimethyl acetal, trichloro acetal, acetaldehyde bromodiethylacetal, acetaldehyde chlorodiethylacetal, dichloro acetaldehyde, trichloro acetaldehyde, acetylacetone, chloroacetone chloroform, acetyl bromide, acetyl chloride, acetyl fluoride, acetic acid bromoethylester, acetic acid chloroethylester, adipaldehyde, adamantyl fluoroformate, 4-amino-4'-dimethylaminobenzene, 5-aminotetrazole, 3-amino-1,2,4-triazole, azobenzene-l-sulfenyl bromide, 1-acetylimidazole, 3-aminophenylboronic acid hemisulfate, N-acetyl-DL-cysteine, N-acetyl-DL-homocysteinethiolactone, 4-amino-phenylmercuric acetate, N-(4-anilino-l-naphtyl)maleimide, azobenzene-2-sulfenyl bromide, 4-azidophenacyl bromide, 1-amino-5-azidonaphthalene, 4-azidoaniline, 4(5)-azidofluorescein diacetate, 5-azido-2-nitrobenzoic acid, 4-azidophenacyl bromide, 4-azidophenyl isothiocyanate, 4-acetamido-4'-isothiocyanatostilbene-2,2'-disulfonic acid, N-(4-aminobutyl)-N-ethylisoluminol, 4-aminofluorescein, 5-aminofluorescein, 7-amino-4-methylcoumarin, 8-anilinonaphthalene-l-sulfonic acid, auramine O, benzonitrile, 4-benzylamino-7-nitrobenzofurazan, benzyl chloroformate, 4-(BOC-2-aminomethyl)phenylisothiocyanate, 5-BOC-2-mercapto-4,6-dimethylpyrimidine, 2-(BOC-oximino)-2-phenylacetonitrile, t-butyl carbazate, (2-bromoctyl) chloroformate, 3-bromopropionic acid, bromoacetic acid, 4-bromophenacyl bromide, 2,3-butanedione, 3-bromo-3-methyl-2-(2-nitrophenylmercapto)3H-indole, N-(4-(2-benzimidazolyl)phenylmaleimide, 4,4'-bis (dimethylamino)-diphenyl carbinol, bromopyruvic acid, N butylmaleimide, omega-bromo-4-nitroacetophenone, benzophenone 3,3',4,4'-tetracarboxylic dianhydride, (+)-biotin 4-nitrophenyster, (+)-biotin 4-nitrophenylester, 2,2'-biquinolone-4,4'-dicarboxylic acid, bis(4-fluoro-3-nitrophenyl)sulfone, 1,5-bis(succinimidooxycarbonyloxy) pentane, 4-(BOC-aminoethyl)phenylisothiocyanate, N-BOC-1,6-diaminohexane, N-(4-(2-benzimidazolyl)phenylmaleimide, 2',4'-bis(di(carboxymethyl)aminoethyl)fluorescein, 4,4'-bis(dimethylamino)diphenyl carbinol, 4-bromomethyl-7-methoxy-courmarin, BOC-L-tyrosine hydrosuccinimide ester, benzaldehyde, 2-amino benzaldehyde, 3-amino benzaldehyde, 4-amino benzaldehyde, 2-bromo benzaldehyde, 3-bromo benzaldehyde, 4-bromo benzaldehyde, 2-chloro benzaldehyde, 3-chloro benzaldehyde, 4-chloro benzaldehyde, 2-hydroxy benzaldehyde, 3-hydroxy benzaldehyde, 4-hydroxy benzaldehyde, benzene sulfinic acid chloride, benzene sulfonic acid chloride, benzene sulfonic acid fluoride, benzoyl bromide, benzoyl chloride, benzoyl fluoride, 2-chloro benzoic acid anhydride, 3-chloro benzoic acid anhydride, 3-chloro benzoyl chloride, 4-chloro benzoic acid anhydride, 4-chloro benzoyl chloride, bioxirane, 1,2-butylene oxide, butyric anhydride, butyryl bromide, bis(2-nitrophenyl)disulfide, (bis(trifluoroacetoxy)iodo)benzene, chloral, chloral alcoholate, chloracetic acid anhydride, chlorodiphenyl acetyl chloride, cumaldehyde, 1,2-epoxy cyclohexane, caprylic anhydride, caprylic chloride, cyclamen aldehyde, 2-chloro-3,5-dinitropyridine, chloro ethanesulfonic acid, 4-chloro-7-nitrobenzofurazan, citraconic anhydride, 2-cyanopyridine, 1,2-cyclohexanedione, 1,1'-carbonyldiimidazole, 1,1'-carbonyldi(1,2,4-triazole), N-cyclohexyl-N'-(2-morpholinoethyl)carbodiimide metho-p-toluenesulfonate, camphorquinone-10-sulfonic acid, chloramine B, chloramine T, N-chlorosuccinimide, cyanuric fluoride, chloranil, chloroethane sulfonic acid, 4-(chlormercuri)benzenesulfonic acid, 4-(chlormercuri)benzoic acid, 4-chloro-7-sulfobenzofurazan, 1-cyano-4-dimethylaminopyridinium tetrafluoroborate, cystamine, cibacron blue 3G-A, carbethoxymethyl isothyocyanate, diphenylethanal, diphenyl acetic anhydride, diphenyl acetyl chloride, diphenyl carbodiimide, 1,2-diacetylbenzene, N,S-diacetylcysteamine, di-t-butyl dicarbonate, N,N-diethyl-2,4-dinitro-5-fluoroaniline, diethyl pyrocarbonate, 4-dimethylamino-benzene-4'-isothiocyanate, 4-dimethylaminoazobenzene-4'-sulfonyl-chloride, 4-(5-dimethylaminonaphthalene-1-sulfonylamino)phenylisocyanate, 5-dimethylamino-1-naphthalenesulfonyl chloride, dimethylmaleic anhydride, 3,5-dimethyl-l-pyrazolyl formamidinium nitrate, 2,4-dinitro-5-fluoroaniline, 2,4-dinitro-1-fluorobenzene, N,N'-di-t-butylcarbodiimide, N,N'-dicyclohexylcarbodiimide, N-(dimethylaminopropyl)-N'-ethyl carbodiimide, 2-ethoxy-1-ethoxycarbonyl 1,2-dihydroquinoline, 1,4-dithioerythritol, (-)1,4-dithio-L-threitol, 1,4-dithio-DL-threitol, diisopropylfluorophosphate, 4-dimethylaminoazobenzene-4'-sulfonylchloride, 5-dimethylamino-1-naphthalenesulfonylchloride, 4-dimethylamino benzaldehyde, diethyl pyrocarbonate, 1,2-diiodoethane, N-(4-dimethylaminoazobenzene-4')iodoacetamide, N-(7-dimethylamino-4-methyl-3-coumarinyl)maleimide, 2,2'-dipheyl-l-picrylhydrazyl, 2,2'-dithiobis(4-methylthiazole), 5,5'-dithiobis(2-nitrobenzoic acid), (3,3',6) 2,2'-dithiobis(5-nitropyridine), 2,2'-dithio dibenzoic acid, 2,2'-dithiodipyridine, 4,4'-diazidostylbene-2,2'-disulfonic acid, diethylenetriamine pentaacetic acid dianhydride, diethylene pyrocarbonate, 1,5-difluoro-2,4-dinitrobenzene, 1,6-diisocyanatohexane, dimethyl adipimidate N-(dimethylaminopropyl)-N'-ethylcarbodiimide, dimethyl 3,3'-dithiodipropionimidate, dimethylpimelinediimidate, dimethylsuberimidate, 3,3'-dithiodipropionic acid bis(N-hydroxy-succinimide ester), dansylcadaverine, dansylhydrazine, 4-(4,6-dichloro-s-triazin-2-ylamino)fluorescein, 4-(5-dimethyl aminonaphthalene-1-sulfonylamino)phenylisocyanate, dimethyloxyrane, 4-dimethylamino-1-naphthyl isothiocyanate, diphenylborinic anhydride, (diacetoxyiodo)benzene, 6-diazo-5-oxo-5,6-dihydro-1-naphthalenesulfonylchloride, 2,3-dimercapto-1-propanol, 2,4-dinitrophenylhydrazine, 1,6-diphenyl-1,3,5-hexatriene, 1-dodecanethiol, epibromohydrin, alpha-epichlorhydrin, beta-epichlorohydrin, epiiodohydrin, epicyanohydrin, ethanesulfonyl chloride, N-ethoxycarbonyl phtalimide, ethyl acetimidate, N-ethylmaleimide, ethyl trifluoroacetate, ethanethiol, ethyl diazoacetate, furfural, 9-fluorenylmethylchloroformate, 5'-(4-fluorosulfonylbenzoyl)adenosine, formaldehyde, 4-formyl-1-methylpyridinium benzenesulfonate, fluorescein mercuric acetate, fluoresceinisothiocyanate, fluoresceinisothiocyanate isomer I, 4-fluoro-3-nitrophenyl azide, N-(3-fluoranthyl)maleimide, fluoresceinamine, 4-fluoro-7-nitrobenzofurazan, fluram, glycolaldehyde, glyoxylic acid, glycolic anhydride, glycidic acid, glycidol, glycinamide, glyoxal, L-glutathione (oxidized), glutaraldehyde, glutaryl chloride, glutaronitrile, 2-hydroxy-5-nitrobenzyl bromide, 6-hydroxy-2-naphthyl disulfide, hexamethylene diisocyanate, 3-(4-hydroxyphenyl)propionic acid N-hydroxysuccinimidester, N-hydroxy sulfosuccinimide, DL-homocysteine thiolactone, 2'-(4-hydroxy phenylazo)benzoic acid, iodo acetic acid, iodo acetamide, iodo acetid acid ethylester, isobutyryl chloride, isonicotin aldehyde, isonicotinic acid anhydride, 2-iminothiolane, N-iodosuccinimide, N-(iodo acetaminoethyl)-l-naphthylamine-5'-sulfonic acid, iodoacetonitrile, iodomethane, 2-iodobenzoic acid, isatin, isophtalaldehyde, lucifer yellow CH, lucifer yellow VS, methoxy acetyl chloride, metaldehyde, 5,5'-methylene disalicyl aldehyde, methan sulfonyl chloride, methyloxiran, maleic anhydride, mersalyl acid, N-methoxy carbonylmaleimide, 2-(2-(2-methoxyethoxy)ethoxy) acetic acid, methyl isothiocyanate, S-methylisbthiourea sulfate, O-methylisothiourea sulfate, O-methylisothiourea hydrogen sulfate, methyl 4-nitrobenzenesulfonate, 2-methoxy-5-nitrobenzyl bromide, 4-maleimidobutyric acid, 6-maleimidocaproic acid, N-maleoyl-beta-alanine, N-(2-mercaptopropionyl)glycine, mercury orange, O-methyl-N-N'-diisopropylisourea, N-methyl maleimide, S-methyl methane thiosulfonate, methyl 4-nitro benzenesulfonate, malononitrile, methyl 4-hydroxybenzimidate, N-methyl mercaptoacetamide, N-methylphenazonium methyl sulfate, O-acetyl mandelyl chloride, methylglyoxal, 2-nitrobenzene sulfenyl chloride, 4-nitrophenyliodoacetate, 2-nitro-5-thiocyanato benzoic acid, 4-nitro-L-tyrosine ethylester, 1-naphthaldehyde, 2-naphthaldehyde, 2-hydroxy-1-naphthaldehyde, alph anaphthyl thiocyanate, beta naphthyl thiocyanate, ninhydrin, 4-nitrophenyl acetate, 4-nitrophenyl formate, N-nitroso-N-methyl-4-toluenesulfonamide, 2-nitrobenzene sulfenyl chloride, l-hydroxy-2-naphthaldehyde, naphtalic anhydride, naphthalene sulfuric acid chloride, naphthosultone, 1-naphthoic acid anhydride, 1-naphthoic acid chloride, 2-naphthoic acid anhydride, 2-naphthoic acid chloride, octanal, 2-oxoglutaric acid, phenoxy acetic anhydride, phenoxy acetyl chloride, phenyl acetic anhydride, phenyl acetyl chloride, o-phenacyl bromide, m-chloro-p-phenacyl bromide, p-chloro-p-phenacyl bromide, p-bromo-p-phenacyl chloride, phenacyl chloride, phenyl maleic acid anhydride, palmitaldehyde, paraldehyde, pentanal, 4-hydroxy pentanal, 3-hydroxy 2-methyl pentanal, 2-oxopentanal, 4-methyl-2-oxopentanal, 4-oxopentanal, 2-phenyl pentane diic acid, 2-phenyl pentane dioic acid anhydride, 3-phenyl pentane dioic acid anhydride, phtaladehyde, phtaldialdehyde, phtaladehydic acid, phtalic acid anhydride, phtalic acid dichloride, propanal, propanal diethylacetal, 2-bromo propanal, 2-chloro propanal, 3-chloro propanal, 2-chloro-2-methyl propanal, 2,3-dibromo propanal, 2,2-dichloro propanal, 2,3-dichloro propanal, 2-phenoxy propanal, 2-phenyl propanal, 3-phenyl propanal, pivaldehyde, propranolonal, propionic anhydride, propionyl bromide, propionyl chloride, phenyl glycidol, phenyl rhodanate, pentafluorophenyl acetate, phenylisothiocyanate, phenylmethanesulfonyl fluoride, phtalonitrile, pyridoxal hydrochloride, pyromellitic dianhydride, 9,10-phenanthrene quinone, phenylglyoxal, phenylmethanesulfonyl fluoride, D-penicillamine, phenylarsine oxide, N,N'-(1,2-phenylene) dimaleimide, N,N'-(1,4-phenylene)dimaleimide, N-phenylmaleimide, N-propylmaleimide, N-(3-pyrenyl)maleimide, polyethylene glycol 600 diacid, phenacyl bromide, photobiotin, 9,10-phenanthrene quinone, pyridine-4-carboxaldehyde, benzyl rhodanate, phenyl rhodanate, p-tolyl rhodanate, rhodamine b isothiocyanate, succinic anhydride, succinic acid monochloride, benzyl sulfonyl chloride, salicylaldehyde, N-succinimidyl-3-(2-pyridylthio) propionate, 2-sulfobenzoic acid cyclic anhydride, 4-sulfophenyl isothiocyanate, N-succinimidyl 3-maleimidobenzoate, N-succinimidyl 4-maleimidobutyrate, N-succinimidyl 6-maleimidocaproate, N-succinimidyl-3-maleimi dopropionate, sebaconitrile, sulforhodamine 101 acid, sulforhodamine 101 acid chloride, styrene oxide, terephthalaldehyde, p-toluenesulfinyl chloride, o-toluenesulfonyl chloride, p-toluenesulfonyl chloride, 3-tosyloxypropyl isocyanate, trifluoroacetic anhydride, 3-trifluoromethylphenyl isocyanate, 2,2,6-timethyl-1,3-dioxin-4-one, 2,4,6-trinitrobenzenesulfonic acid, triethoxonium tetrafluoroborate, trimethyloxonium tetrafluoroborate, 1-thioglycerol, thioglycolic acid tributylphosphine, N-tosyl-L-lysine chloromethylketone, tosyl-L-phenylalanine chloromethylketone, thiodiethylene glycol, toluylene-2,4-diisocyanate, tetramethylrhodamine B isothiocyanate, 6-(4-toluidino)-2-naphthalenesulfonic acid, 2,2,2-trichloroethanol, toluene sulfonic acid, vanilin, veratrylaldehyde, valeric aldehyde, valeryl chloride, m-xylylene diisocyanate, N-acetyl-beta-glucosamine phenylisothiocyanate, alpha-glucopyranosylphenyl isothiocyanate, beta-glucopyranosylphenyl isothiocyanate, beta-galactopyranosylphenyl isothiocyanate and alpha-mannopyranosylphenyl isothiocyanate.

Functionalized RDPs can be obtained by choosing a conjugand with the proper functional group for radio-derivatization. For example, nucleophilized RDPs can be obtained by using nucleophile-containing conjugands. This principle is shown in Figure 2. When polystyrene is radio-derivatized in the presence of aromatic amines, such as p-aminophenol, DL-2-amino-1 (p-hydroxyphenyl)-ethanol-l or benzyl esters of amino acids, the resulting RDPs can be used for the coupling of carboxyl-containing compounds with carbodiimide.

The choice of conjugand to make a functionalized RDP depends on the particular functionalization desired. For example, aromatic amines can be used if a functional group capable of being diazotized is desired. When a nucleophile-functionalized RDP is desired, compounds that contain nucleophilic groups such as amino, hydroxyl or mercapto groups should be used as the conjugand. The preferred conjugands in this class are amines which contain aromatic structures, but not necessarily aromatic amines. D Examples of these preferred conjugands are anilin and its deriviatives (e.g., m-phenylene diamine), amino phenols (e.g., p-amino phenol), naphthylamines (e.g., 1,2,naphthylene diamine) aromatic esters of aminoacids (e.g., L-serine benzyl ester) and compounds that contain different nucleophile such as 2-amino 1(4-hydroxyphenyl)ethanol-1 (AHE). Specific examples of suitable nucleophile-containing conjugands for preparing nucleophile-functionalized RDPs are: thionalide, thioglycolic acid anilide, p-acetyl phenol, o-mercapto anilin, m-mercapto anilin, p-mercapto anilin, 1-hydroxy anthracene, 2-hydroxy anthracene, 9-hydroxy anthracene, 3-hydroxy azobenzene, 4-hydroxy azobenzene, m,m'-methylene diphenol, p,p'-methylene diphenol, 1,5-dihydroxy naphthalene, 1,6-dihydroxy naphthalene, 1,7-dihydroxy naphthalene, 1,8-dihydroxy naphthalene, 2,6-dihydroxy naphthalene, 2,7-dihydroxy naphthalene, 1-hydroxy naphthalene, 2-hydroxy napthalene, 2-hydroxy phenanthrene, 3-hydroxy phenanthrene, 9-hydroxy phenanthrene, phenol, 2-bromophenol, 3-bromophenol, 4-bromophenol, 3-bromo-5-chlorophenol, 4-bromo-2-chlorophenol, 2-chlorophenol, 3-chlorophenol, 4-chlorophenol, 2,4-dibromophenol, 2,6-dibromo phenol, 2,3-dichlorophenol, 2,4-dichlorophenol, 2,5-dichlorophenol, 2,6-dichlorophenol, 3,4-dichlorophenol, 3,5-dichlorophenol, 2-iodophenol, 3-iodophenol, 4-iodophenol, 2-mercaptophenol, 3-mercaptophenol, 4-mercaptophenol, 2-pyridol, 3-pyridol, 4-pyridol, saligenin, alpha-3-dihydroxy toluene, alpha,2-dihydroxy toluene, alpha,4-dihydroxy toluene, 2,4-dihydroxy toluene, 2,5-dihydroxy toluene, 3,5-dihydroxy toluene, 2-hydroxy toluene, 3-hydroxy toluene, 4-hydroxy toluene, tyrosine, and DL-2-amino-1(4-hydroxyphenyl)-ethanol-1.

Specific examples of suitable amino group-containing conjugands for preparing functionalized RDPs that are reactive with carboxyl-containing compounds in the presence of activators, such as carbodiimides are: n-butylamine, s-butylamine, t-butylamine, putrescine, cyclohexylamine, ethylamine, ethylene diamine, hexamethylene diamine, 1-amino pentane, 2-amino pentane, 3-amino pentane, cadaverin, phenylalanine, propylamine, isopropylamine, amphetamine, 1,2-propane diamine, trimethylene diamine, DL-alanine, beta alanine, benzylamine, m-bromo benzylamine, p-bromo benzylamine, and triethylene tetramine.

The preferred amino group-containing conjugands suitable for coupling of various carboxyl compounds in the presence of an EDC activator are DL-2-amino-1(4-hydroxyphenyl)-ethan-1-ol, p-aminophenol, 3,4-diamino pyridine, 1-alanine benzyl ester, L-serine benzyl ester (See Example 4, Figure 2).

The term activators as used herein refers to those types of substances suitable for activating carboxyl groups for peptide synthesis, which include mixed anhydride types, active ester types, hydroxylamine types and various coupling reagents. (See M. Bodanszky: Principles of Peptide Synthesis, Springer Verlag, New York, 1984). In addition to being reactive with amino groups and suitable for peptide bond formation, these activators can activate carboxyl groups to react with other functions such as SH and OH. Specific examples of suitable activators are: acyl chlorides such as valeryl chloride, phosphorous acid chlorides, arsenous acid chlorides, phosphoric acid chlorides, pyridinium phosphoryl chlorides, acylphosphonium salts, sulfuric acid derivatives, thiol acids, leuchs' anhydrides, active esters such as phenylesters, nitrophenylesters, dinitrophenylesters, methoxyphenylesters, chlorophenylesters, fluorophenylesters, reactive hydroxylamine derivatives such as hydroxyphthalamide esters, N-hydroxysuccinimide esters, N-hydroxypiperidine esters; coupling reagents: specifically carbodiimides such as dicyclohexyl carbodiimide, ethyl-dimethylaminopropyl carbodiimide (EDC), 1-ethoxycarbonyl-2-ethoxy-1,2-dihydroquinoline (EEDQ), l-isobutyoxycarbonyl-2-isobutyloxy-1,2-dihydroquinoline (IIDO), diphenylketenes, acyloxy-phosphonium salts. Auxiliary nucleophiles can be used as additives in coupling reactions (primarily with carbodiimides) such as N-hydroxysuccinimide, 1-hydroxybenztriazole, 3-hydroxy-3,4-dihydro-1,2,3,-benztriazin-4-one (HOObt).

An important group of RDPs are those obtained with conjugands that are aromatic amines. Preferably, aromatic amines such as phenylene diamines, aminopyrimidine, naphthylamine or 5-I-2-aminopyrimidine yield RDPs that can be diazotized wherein the diazonium salt derivative can be further used for coupling suitable molecules such as proteins with phenol side groups (Example 5, Table 4). The preferred aromatic amines found particularly suitable for producing diazotizable RDPs suitable for yielding relatively high coupling of RNase or hemoglobin are 1-naphthylamine, 5-I-2-aminopyrimidin, 1,2-naphthylene diamine, 1-5-naphthylene diamine, m-phenylene diamine, and 2,6-diamino pyridine (from Table 4).

Specific examples of suitable aromatic amines that can be used for producing functionalized RDPs or diazotizable RDPs are: 2-amino azobenzene, 3-amino azobenzene, 4-amino azobenzene, 2,2'-diamino azobenzene, 2,4-diamino azobenzene, 4,4'-diamino azobenzene, 1-chloro-2,4-diaminobenzene, 1-chloro-3,5 diaminobenzene, 2-chloro1,3-diaminobenzene, 2-chloro-1,4-diaminobenzene, 4-chloro-1,2-diaminobenzene, 1,3-diamino benzene, 1,4-diamino benzene, orthanilic acid, methanilic acid, sulfanilic acid, 3,5-diamino benzenesulfonic acid, 2-amino benzimidazole, anthranilic acid, 3-amino benzoic acid, 4-amino benzoic acid, 2,4-diamino benzoic acid, 2,5-diamino benzoic acid, 3,4-diamino benzoic acid, 3,5-diamino benzoic acid, 2-amino benzophenone, 3-amino benzophenone, 4-amino benzophenone, 2,2'-diamino benzophenone, 3,3'-diamino benzophenone, 2-amino biphenyl, 3-amino biphenyl, 4-amino biphenyl, 2,2'-diamino biphenyl, 2,4'-diamino biphenyl, 3,3'-diamino biphenyl, 4,4'-diamino biphenyl, o-benzhydraniline, m-benzhydraniline, p-benzhydraniline, 4,4'-diaminoditan, 4,4'-diaminotritan, pararosaniline, 1-naphthylamine, 2-naphthylamine, 1,2-diamino naphthalene, 1,5-diamino naphthalene, 2,6-diamino naphthalene, 1,7-diamino naphthalene, 1,8-diamino naphthalene, 2,6-diamino naphthalene, 3-amino phenanthrene, 9-amino phenanthrene, o-hydroxy aniline, m-hydroxy aniline, p-hydroxy aniline, o-acetyl aniline, m-acetyl aniline, p-acetyl aniline, p-amino phenacyl chloride, 2-amino acridine, 3-amino acridine, 4-amino acridine, 9-amino acridine, 3,6-diamino acridine, aniline, 3-acetamido aniline, 4-acetamido aniline, 2-bromo aniline, 3-bromo aniline, 4-bromo aniline, 2-chloro aniline, 3-chloro aniline, 4-chloro aniline, 2,3-dibromo aniline, 2,4-dibromo aniline, 2,5-dibromo aniline, 2,6-dibromo aniline, 3,5-dibromo aniline, 2,3-dichloro aniline, 2,4-dichloro aniline, 2,5-dichloro aniline, 3,4-dichloro aniline, 3,5-dichloro aniline, o-butyl aniline, p-butyl aniline, 4-dimethylamino aniline, o-ethyl aniline, m-ethyl aniline, p-ethyl aniline, o-phenetidine, m-phenetidine, p-phenetidine, 2-phenoxy aniline, 3-phenoxy aniline, 4-phenoxy aniline, 1-amino anthracene, 2-amino anthracene, 9-amino anthracene, 1,2-diamino anthraquinone, 1,3-diamino anthraquinone, 1,4-diamino anthraquinone, 1,5-diamino anthraquinone, 1,6-diamino anthraquinone, 1,7-diamino anthraquinone, 1,8-diamino anthraquinone, 2,3-diamino anthraquinone, 2,6-diamino anthraquinone, 2,7-diamino anthraquinone, 5-iodo-o-toluidine, 4-iodo-o-toluidine, 2-iodo-m-toluidine, 6-iodo-m-toluidine, 5-iodo-m-toluidine, 3-iodo-p-toluidine, 2-iodo-p-toluidine, 4-iodo-m-toluidine, 2,3-diamino toluene, 2,4-diamino toluene, 2,5-diamino toluene, 2,6-diamino toluene, 3,4-diamino toluene, 3-5-diamino toluene, and tyramine.

In addition to producing diazotizable RDPs, the aromatic amines listed above can also be used to produce RDPs that have other reactive properties, namely, RDPs that are reactive with carboxyl-containing compounds in the presence of activators, RDPs that are reactive with bi-functional reagents and those that are reactive with nucleophile-functionalized RDPs. In addition to amino groups, other relatively radio resistant nucleophiles or nucleophiles that survive irradiation during radio-sterilization in sufficient numbers to be used for coating the plate with adequate densities of nucleophiles, for example SH or OH, can be linked to polymers to produce nucleophilized RDPs.

Conjugands that are carbohydrates or derivatives thereof can be used to derivatize polymers under irradiation and produce glycophase RDPs. The term glycophase RDP as used herein refers to a solid support carrying vicinal diols, usually in the form of a covalently attached carbohydrate to glass (distributed by Corning, Pierce), to Sephadex, Sepharose (distributed by Pharmacia) or other similar products. The glycophase RDPs can be activated with cyanogen bromide as shown in Ceska et al., J. Allerg. Clin. Immunol. Vol. 49, 1972, pp. 1-9 or with periodate as shown in Varga, Methods Enzymol., Vol. 112, 1985, pp. 259-269 or with other suitable activating substances. The particularly preferred conjugands used for producing glycophase RDPs are phenol-alpha-D-galactosid, phenol-beta-D-galactosid and phenol-beta-D-glucosid. Additional suitable conjugands which can be used for producing glycophase RDPs include: 2-acetamido-4,6-benzylidene-2-deoxy-beta-glucopyranose, N-acetyl-beta-glucosamine naphthol, p-aminobenzyl-1-thio-2-acetamido-2-deoxy-beta-glucopyranoside, p-aminophenyl-2-acetamido-2-deoxy-1-thio-beta-glucopyranoside, p-aminophenyl-alpha-fucopyranoside, p-aminophenyl-alpha-galactopyranoside, p-aminophemyl-beta-galactopyranoside, p-aminophenyl-alpha-glucopyranoside, p-aminophenyl-beta-glucopyranoside, o-aminophenyl-alpha-glucuronide, p-aminophenyl-1-thio-beta-glucuronide, p-aminophenyl-beta-lactopyranoside, p-aminophenyl-alpha-mannopyranoside, p-aminophenyl-beta-thiofucopyranoside, p-aminophenyl-l-thio-beta-galactopyranoside, p-aminophenyl-1-thio-beta-glucopyranoside, p-aminophenyl-l-thio-beta-xylopyranoside, p-aminophenyl-beta-xylopyranoside, D-amygdalin, N-benzoyl-alpha-glucosamine, benzyl 2-acetamido-6-O-(2-acetamido-2-deoxy-beta-glucopyranosyl)-2-deoxy-alpha-glucopyranoside, benzyl 2-acetamido-2-deoxy-alpha-galactopyranoside, benzyl 2-acetamido-2-deoxy-3-O-beta-galactopyranosyl-alpha-galactopyranoside, benzyl 2-acetamido-2-deoxy-alpha-glucopyranoside, benzyl-2-acetamido-2-deoxy-beta-glucopyranoside, benzyl 2-acetamido-2-deoxy-3-O-beta-glucopyranoside, benzyl-N-CBZ-glucosamidine, benzyl-4-O-beta-galactopyranosyl-beta-glucopyranoside, 4,6-O-benzylideneglucopyranoside, 4,6-O-benzylideneglucose, 6-bromo-2-naphthylgalactopyranoside, 6-bromo-2-naphthylglucopyranoside, 6-bromo-2-naphthylglucopyranoside, n-butyl 4-O-galactopyranosyl-beta-glucopyranoside, 6,7-dihydroxycoumarin-6-glucoside, n-dodecyl beta-glucopyranoside, n-dodecyl beta-maltoside, esculin, fluorescein di-(beta-galactopyranoside), beta-galactose naphthol, heptanoyl-N-methylglucamide, n-heptyl beta-glucopyranoside, hesperidin, 2-hexanedecanoylamino-4-nitophenyl-beta-galactopyranoside, 2-hexadecanoylamino-4-nitrophenyl-beta-glucopyranoside, n-hexyl-beta-glucopyranoside, 8-hydroxyquinoline-beta-glucopyranoside, 8-hydroxyquinoline glucuronide, mandelonitrile-beta-glucoside, mandelonitrile glucuronic acid, alpha-naphthyl-alpha-galactopyranoside, alpha-naphthyl-beta-galactopyranoside, beta-naphthyl-beta-galactopyranoside, beta-naphthyl-alpha-glucopyranoside, beta-naphthyl-beta-glucopyranoside, beta-naphthyl-alpha-glucoside, alpha-naphthyl-beta-glucuronide, naringin, n-nonyl glucoside, n-nonyl glucopyranoside, octadecylthioethyl 4-O-alpha-galactopyranosyl-beta-galactopyranoside, octanoyl-N-methylglucamide, n-octyl alpha-glucopyranoside, n-octyl-beta-glucopyranoside, phenolphthalein glucuronic acid, phenolphthalein mono-beta-glucosiduronic acid, phenolphthalein mono-beta-glucuronic acid, phenyl-2-acetamido-2-deoxy-alpha-galactopyranoside, phenyl-2-acetamido-2-deoxy-alpha-glucopyranoside, alpha-phenyl-N-acetylglucosaminide, beta-phenyl-N-acetylglucosaminide, phenylethyl-beta-galactoside, phenyl-beta-galactoside, phenyl-beta-galactopyranoside, phenyl-alpha-glucopyranoside, phenyl-beta-glucopyranoside, phenyl-alpha-glucoside, phenyl-beta-glucoside, phenyl-beta-glucuronide, phenyl-alpha-mannopyranoside, phenyl-beta-thiogalacto-pyranoside, phenyl-beta-thiogalactoside, and salicin.

By using carboxyl-containing compounds as the conjugand, a carboxylated RDP can be produced, which can be further activated with carbodiimides or other suitable activators recited herein. For the activation of solid phase-bound carboxyls, see Sott, L. T., et al., (J. Am. Chem. Soc., Vol. 99, 1977, pp. 625-626). The term carboxyl-containing compound as used herein means conjugands that contain at least one carboxyl group and at least one aromatic structure in the same molecule, but not necessarily aromatic carboxyls. Examples of the particularly preferred carboxyl-containing conjugands are benzoic acid, naphthoic acid and its derivatives, phenyl acetic acid and phenyl naphthoic acid and its derivatives. Additional carboxyl-containing compounds which can serve as conjugands for producing carboxylated RDPs include: acetic acid, o-bromo-toluic acid, m-bromo-toluic acid, o-chloro-toluic acid, m-chloro-toluic acid, p-chloro-toluic acid, homogentisic acid, diphenyl acetic acid, benzylic acid, phenyl glycolic acid, o-hydroxy-alpha-toluic acid, m-hydroxy-alpha-toluic acid, p-hydroxy-alpha-toluic acid, thioglycolic acid, methoxyphenyl acetic acid, 1-naphthoxy acetic acid, 2-naphthoxy acetic acid, 1-naphthalene acetic acid, N-benzoyl aspartic acid, o-azobenzoic acid, m-azobenzoic acid, p-azobenzoic acid, phthalic acid, isophthalic acid, terephthalic acid, 2-amino-1,3-benzene dicarboxylic acid, 4-amino-1,3-benzene dicarboxylic acid, 5-amino-1,3-benzene dicarboxylic acid, 2-amino-1,4-benzene dicarboxylic acid, 4-amino-1,4-benzene dicarboxylic acid, 5-amino-1,4-benzene dicarboxylic acid, hemimellitic acid, trimellitic acid, trimesic acid, benzoic acid, 2-chloro-benzoic acid, 3-chloro-benzoic acid, 4-chloro-benzoic acid, anilino acetic acid, N-benzoyl leucine, N-benzoyl lysine, 1-naphthoic acid, 2-naphthoic acid, 1,2-naphthalene dicarboxylic acid, 1,4-naphthalene dicarboxylic acid, 1,5-naphthalene dicarboxylic acid, 1,6-naphthalene dicarboxylic acid, 1,7-naphthalene dicarboxylic acid, 1,8-naphthalene dicarboxylic acid, 2,3-naphthalene dicarboxylic acid, 2,6-naphthalene dicarboxylic acid, 2,7-naphthalene dicarboxylic acid, 2-phenyl pentane dioic acid, valerianic acid, 1-phenanthrene carboxylic acid, 2-phenanthrene carboxylic acid, 3-phenanthrene carboxylic acid, phthalic acid, proline, propionic acid, 2,2-diphenyl propionic acid, 2,3-diphenyl propionic acid, 3,3-diphenyl propionic acid, 2,3-diphenyl-2-hydroxy propionic acid, 3,3-diphenyl-2-hydroxy propionic acid, 3,3-diphenyl-3-hydroxy propionic acid, benzoyl glycolic acid, alpha-hydroxyhydrocynnamic acid, 3-hydroxy-2-phenyl propionic acid, 3-hydroxy-3-phenyl propionic acid, melilotic acid, 2-methyl-3-phenyl-propionic acid, 3(1-naphthyl)-propionic acid, 3(2-naphthyl)-propionic acid, phenyl pyruvic acid, benzoyl acetic acid, 2-phenoxy propionic acid, 3-phenoxy propionic acid, 2-phenyl propionic acid, 3-phenyl propionic acid, picolinic acid, nicotinic acid, isonicotinic acid, quinolinic acid, lutidinic acid, dipicolinic acid, dinicotinic acid and benzyl succinic acid.

Protein modifying reagents that are known in the art (See Lundblad, L.L., Noyes, C.M.: Chemical reagents for protein modification, CRC Press, Boca Raton, 1984) can serve as conjugands for producing RDPs that are nucleophile-reactive polymers. The preferred protein-modifying reagents are those protein modifying reagents that contain at least one protein reactive group and at least one aromatic structure such as Bolton-Hunter reagent, OPA, TNBSA, Dansyl chloride, Dabsyl chloride, DABITC, SBF-chloride, Ellman's reagent, p-hydroxyphenylglyoxal, BTI, Ninhydrin, (See Pierce 1988 Handbook for the abbreviations) or fluorescamin, FITC, RITC FLUOS, RESOS, RHODOS, and Resornfin from Sigma Co. Additional suitable protein-modifying reagents are previously recited herein under conjugands for making reactive RDPs.

Bi-functional reagents can be used to react with functionalized RDPs, specifically nucleophile-functionalized RDPs and RDPs formed from conjugands that contain amino groups or water. The term bi-functional reagent as used herein refers to cross-linking reagents that are capable of combining or bridging the RDP with at least one ligand. The term bi-functional reagents includes polyfunctional reagents or "cross-linking" reagents that contain multiple functional groups. The preferred bi-functional reagents are EDC, BDE and TDI (See Table 5). Additional examples of bi-functional or cross-linking reagents used in this invention include: dialdehydes, preferably glutaraldehyde, diisothiocyanates, preferably phenylene 1,4-diisothiocyanate, diisocyanates, preferably toluylene 2,4-diisocyanate, bis diazo benzidines, bis diazo-o-anisidine, biepoxides and chloro-5-triazines.

An important aspect of this invention is that there is substantial overlap between the different classes of conjugands used to make the different classes of RDPs. For example, the conjugand p-aminophenol is both an aromatic amine which can be diazotized and it can also be turned into a quinone, which explains why this particular conjugand can be used to produce functionalized RDPs and reactive RDPs. Aromatic amine conjugands are diazotizable and, at the same time, they are nucleophile-containing; thus, in addition to diazotization, they are functionalized, suitable for coupling with bifunctional reagents and activators. Similarly, some of the reactive RDPs can also serve as functionalized RDPs.

It has been found that conjugands containing aromatic structures yield higher levels of incorporation into polystyrene than aliphatic conjugands. (Example 1, Table 1). Accordingly, aromatic compounds are preferred conjugands for radio-derivatization of polystyrene. In principle, this need not apply to other polymers such as polyethylene.

One of the simplest RDP products in this invention is formed with water as the conjugand. As shown in Table 2, relatively large amounts of water incorporate into polystyrene during radio-derivatization. The reactivities of the polystyrene-water RDP are suggestive of the formation of reactive nucleophiles (Example 7-11). They are able to form covalent bonds with DNP-aminoacids in carbodiimide-mediated reactions, and the immobilized ligand can be used in highly reproducible immunoassays (Example 22). Additionally, water-RDPs are also formed as side products when radio-derivatization is carried out in aqueous solutions.

The RDPs produced in this invention have many applications. They can be used, for example, "as such," without further modifications, for example, as substrates for cell cultures with improved adherence and growth-supporting properties, or as sorbents for chromatography using, for example, radio-aromatized, radio-alkylated, radio-carboxylated, particles. These RDPs can be especially useful when non-suppressible materials are needed, for example, for HPLC and high-performance affinity chromatography.

Reactive and functionalized RDPs have a number of applications. A large number of molded polymers, mainly polystyrene and its co-polymers, can be produced to be used as microtiter plates, tissue culture plates or pipettes. The adsorptive binding and/or reactive characteristics of these products can be favorably modified by radio-derivatization, followed by immobilization of suitable ligands. In comparison with the methods currently in use, the present method of radio derivatization has quite obvious advantages when transparent support materials are needed (microtiter plates, tissue culture labware) and when these products are exposed to high energy irradiation for the purpose of sterilization. In this case, RDPs with favorable adsorptive and/or covalent binding capacities can be obtained with relatively little extra manufacturing efforts other than irradiation used for radio-sterilization. The resulting RDPs can then be used for several applications 1) The method is generally suitable for the production of polymeric surfaces with covalently attached ligands for binding assays, including immunoassays. The method can be especially advantageous for colorimetric assays, since the optical transparency of RDPs is unimpaired. In addition, the method can be favorably considered for other types such as radiometric or fluorometric assays because of the low-background noise supports obtainable by radio-derivatization. 2) RDPs can be used for the covalent attachment of adhesions-molecules, antibodies, or biologically active molecules for mass cultivation of cells and panning. 3) Radio-derivatized polymers in foil and/or sheet form can be used for blotting and hybridizations. 4) Radio-derivatized polymer particles (such as polystyrene "latex" beads) can be used for the preparation of affinity sorbents, ion-exchangers or hydrophobic sorbents, that are insensitive to bases and non-suppressible, thus being suitable for HPLC columns; "reactive polymers" for organic synthesis; solid support for peptide-, oligonucleotide- and oligosaccharide synthesis; immobilized enzymes for "enzyme reactors"; immobilized antigens for the production of vaccines; immobilized transporters. 5) RDPs in liquid, gel or granular forms can be used as drug carriers. 6) The method of radio-derivatization can be considered for the preparation of molecular printed circuits in the manufacturing of microchips.

The types of molecules, particles or substances that are immobilized in the applications described above are referred to herein as ligands. The ligands in the present invention can be organic or inorganic substances. To exemplify and without any limitation thereof, additional ligands which can be immobilized, fixed or further linked onto the radio-derivatized polymers produced in this invention include cofactors, coenzymes, chelators, ions, hormones, drugs, dyes, lipids, lectins, carbohydrates, nucleic acids, enzymes, haptens, antigens, allergens, antibodies, viruses, microorganisms, eukaryotic cells and representative components thereof.

In the immobilization of ligands on RDPs with cross-linkers and activating agents, the standard methods of coupling of ligands and proteins to carbohydrate-based (e.g., "Sepharose") or polyacrylamide, and other (e.g., inorganic) supports are generally known in the art. The following main types of covalent coupling methods can be considered, depending on the nature of the functionalized RDP and the ligand to be immobilized: a) Carboxyl-containing molecules can be converted into nucleophile-reactive derivatives (i.e., with carbodiimide) and exposed to nucleophilised RDP (Example 4, Figure 2). The same method can be used with proteins (Example 6, Table 5). Considering this general method, other reagents, such as symmetric or mixed acid anhydrides, Leuch's anhydrides, acid azides, or reactive hydroxylamine derivatives can be used. The yield of these types of coupling reactions can be increased with the inclusion of "helper nucleophiles" such as l-hydroxybenzotriazol, other benzotriazine derivatives and hydroxysuccinimide, etc.; b) Diazonium salt-reactive (e.g., phenol- RDPs can be used for coupling diazotized molecules; c) Homo- or heterobifunctional cross-linkers can be used that are able to react with functional groups present on the RDPs and the molecules to be immobilized (Table 2); d) RDPs can be further modified with "spacers" using Leuch's anhydrides, beta-alanin, oligoglycine, etc., using e.g., amino-functionalized RDPs and amino-blocked spacers. The amino-spacer-RDP can be obtained after deblocking.

This invention is not limited to the coupling methods discussed above, as numerous other methods known in the art can be used for coupling molecules to functionalized RDPs.

The resulting RDPs formed by the conjugand and the polymer and the final RDPs linked to ligands can be identified by radiometry by using radiolabelled conjugands (Example 1, Table 1). Radio-labelled ligands or ligand-binding proteins (Figure 2, Tables 3-5) can be used to identify the ligand-binding protein to the RDP. Other methods known in the art can also be used.

Although all types of polymers containing radiolysable bonds can be used for radio-derivatization, the preferred embodiment of this invention and all the examples described below have been carried out with polystyrene. The conjugands were added to polystyrene (PS) microtiter plates (non-sterile unirradiated, obtained from the Greiner GmbH, Kremsmunster, Austria; 100 µl/well) 100 mM (unless noted otherwise) in water (pH 9, NaOH). The plates were sealed with adhesive foil (Nunc® No 236269) and hermetically packed with a polyethylene foil heat sealer (distributed by Krups Co., FRG), then exposed to a gamma source as described above. Nonirradiated (control) plates were kept at room temperature for the same length of time with the same substances as the irradiated ones and subsequently processed the same way as the irradiated plates. The solutions were removed from the plates by vigorous shaking and the plates were washed in sequence: 10 times with tap water, then 200 µl NaHCO₃ (0.1 M) was added and kept at room temperature for 5 minutes, the solution was removed and 200 µl KCl (2 M in glycine buffer (0.2 M, pH3) was added and kept at room temperature for 15 minutes; the last two washing steps were repeated four times followed by washing 10 times with tap water and twice with distilled water; the plates were used immediately for binding studies or they were stored in a vacuum at room temperature. Under these conditions there was no significant loss of reactivity in one month.

Conjugands that are known in the art to hydrolyze, or otherwise react with water under conditions of radio-derivatization, necessitate an alternative washing procedure. The plates should be washed five times with anhydrous formamide following radio-derivatization, instead of the above aqueous washing procedures, and used immediately for immobilization of molecules. General examples of conjugands that would hydrolyze under the conditions of radio-derivatization (in acqueous alkaline solution) are acid anhydrides, acid halides, isothiocyanantes, carbodiimides, oxyranes and nitro-aromatic halides.

Conjugands that hydrolyze in water are used preferably in undiluted form when the conjugand is liquid at room temperature. Both liquid and solid hydrolyzable conjugands can be used in solution in anhydrous formamide or other solvents, such as DMSO, which do not dissolve the polymer, i.e., polystyrene, and do not lyse the conjugand.

Generally, conjugands that do not hydrolyze are used preferably in aqueous, alkaline solution, but can also be used in solution with solvents other than water, provided such solvents do not dissolve the polymer (i.e., polystyrene) and do not lyse the conjugand, such as formamide. Non-hydrolyzable conjugands that are in liquid state at room temperature can also be used in undiluted form. The use of liquid non-hydrolyzable conjugands in undiluted form is preferred when the conjugand has low solubility in water and/or formamide. Among the examples disclosed herein, 1,3-diamino-2-hydroxy propane was used in undiluted form.

The uptake of radiolabeled conjugands was measured using the same procedure of radio-derivatization discussed above with the following modifications: 0.1-3 µCi of radiolabeled conjugand was added as a tracer to the unlabeled substance and following irradiation and the washing steps the wells of microtiter plates were cut out with a red-hot razor blade, solubilied with Bioflour® (NEN) emulsifier cocktail and counted in a liquid scintillation counter (LKB/Wallac® 1215).

Irradiation was performed using A⁶⁰Co. gamma-source (ca. 17 000 Ci) with an intensity of 0.1-0.2 Mrad/h to a total dose of 1-15 Mrad, preferably 3-5 Mrad, at normal atmospheric pressure, at room temperature. Exclusion of oxygen during irradiation generally has a favorable effect on the reactivity of RDPs. However, even if irradiation in the presence of oxygen yields RDPs with lower reactivity, they are sufficiently reactive for the immobilization of ligands for immunoassays, See Example 22. Although the above gamma-source type of irradiation was used in the Examples herein, any type of high-energy irradiation (i.e. electron-radiation) that is capable of radiolysing the polymer and/or conjugand can be used in this invention.

Ethyldiethylaminopropyl-carbodiimide or EDC-mediated coupling was performed for small molecules by adding 50 pl solutions (2 mM in NaCl, 0.1 M, pH 5, or pH 3 for DNP-Gly) of the molecules to be coupled to the wells of radio-derivatized microtiter plates followed by 50 µl EDC solution (2 mg/ml in NaCl, 0.1 M, pH 5 or pH 3 for DNP-Gly). The coupling was allowed to proceed at room temperature for two hours in the dark with light sensitive materials; non-bound material was removed by washing and the uptake of labeled compounds was measured.

For EDC-mediated coupling of proteins 1 mg/ml protein solutions with approximately 100,000 cpm trace[¹²⁵I] labeled proteins were used at room temperature for two hours in the dark with light sensitive materials and with 10 mg/ml EDC solution, pH adjusted to 5. The non-bound proteins were removed as described above except that washing solutions were supplemented with 0.05% Tween 20 and PBS was used instead of the KCl/glycine solution. The radioactivity was counted in a gamma counter (LKB/Wallac CliniGamma). The proteins were labeled with ¹²⁵I using the "Enzymobead" method of Bio Rad, Richmond, California.

Diazo-coupling was obtained by the following procedure: Radio-derivatized (and control) plates were precooled on ice-water for 30 minutes; ice cold NaNo₂ solution (5 mg/ml in 2N HCl) was added and kept on ice-water for one hour. The liquid was removed and the plate was washed three times with ice cold water. 100 µl protein solution [1 mg/ml (+ 0.1 µCi trace-labeled protein) in borate buffer (0.1 M, pH 8.5)] was added to the wells and the coupling was allowed to take place overnight at 4°C. Non-bound protein was removed; the plates were washed and the uptake of proteins was determined as described above.

Coupling with cross-linkers was obtained by pretreating the radio-derivatized plates with bifunctional reagents [100 µl/well, 25 mg/ml in phosphate buffer (0.2 M, pH 7.2)] for two hours at room temperature. The reagents were removed and the protein solutions containing trace-labeled proteins were added. Coupling was allowed to continue overnight at 4°C. The non-bound proteins were removed and the amount of bound substance was measured as described above.

The above methods and materials can be altered without departing from the spirit and scope of the invention or sacrificing its advantages. The following Examples merely illustrate and do not limit the invention.

Example 1
Commercially available radiochemicals (listed in Table 1) in amounts of 2-5 µCi/50 µl/well, diluted with 1-100 mM cold substance in aqueous solutions (pH 9, NaOH) were added to non-irradiated (Greiner® No. 651 101) microtiter plate wells and the wells were sealed with plate adhesive foil (Nunc® No. 236 269). In addition, the plates were sealed individually with a polyethylene foil heat sealer and irradiated with ⁶⁰Co gamma-source (Ca. 17000 Ci) with an intensity of 0.1-0.2 Mrad/h to a total of 1-15 Mrad, preferably 3-5 Mrad, at normal atmospheric pressure at room temperature. The non-irradiated (control) plates containing the same substances were kept at room temperature for the same length of time as the irradiated plates and subsequently processed the same way as the irradiated plates. Following irradiation the contents of the wells were removed with adsorbing paper and the plates were washed in the following sequence: 10 times with tap water, then 200 µl NaHCO₃ (0.1 M) was added and kept at room temperature for 5 minutes, the solution was removed and 200 µl KCl (2 M in glycine buffer) (0.2 M, pH3) was added and kept at room temperature for 15 minutes. The last two washing steps were repeated four times followed by washing 10 times with tap water and twice with distilled water. The plates were dried on air; the wells were cut out with a red-hot razor blade, solubilized with "Biofluor" (NEN) emulsifier cocktail and counted in an LKB/Wallac® 1215 liquid scintillation counter. The results are shown in Table 1.

**TABLE 1:**

| Radiation-mediated uptake of radiochemicals by polystyrene | | | |
|---|---|---|---|
| SUBSTANCE | CONDITIONS | | F(RC)⁺ |
| | non-irradiated | irradiated | |
| | uptake nmoles/well (S.D.) | | |
| [3H]Na-acetate | 2.60(0.36) | 3.90(1.02) | 1.5 |
| [2-3H]glycine | 0.80(0.09) | 2.80(0.70) | 3.5 |
| L-[4,5-3H] lysine | 2.90(0.27) | 3.77(0.85) | 1.3 |
| [5,6(n)-3H]prostaglandinE1 | 1.55(0.13) | 1.86(0.46) | 1.2 |
| [1,4(n)-3H] putrescine | 0.25(0.06) | 2.78(0.75) | 11.1 |
| 3-[5(n)-3H]indolylacetate | 1.10(0.22) | 2.30(0.67) | 2.1 |
| Benzyl[14C]penicillin | 0.60(0.06) | 11.22(2.86) | 18.7 |
| [7-14C]benzoic acid | 0.75(0.09) | 38.85(5.5) | 51.8 |
| [7-14C]salicylic acid | 0.80(0.14) | 23.20(0.67) | 29.0 |

| | | | |
|---|---|---|---|
| ⁺F(RC): factor of radio-derivatization = uptake(irradiated)/uptake(non-irradiated) | | | |

Example 2: Polystyrene-water RDP
To anhydrous formamide, dimethylsulfoxide or to H₂O, tritiated water (sp. RA ca 2000 Ci/ml) was added in increasing concentrations; plates containing 20 µl/well solutions were irradiated, processed and covalently bound [3H] was measured as described in Example 1; non-irradiated control plates were exposed to the same solutions for the same length of time as irradiated plates. The results are shown in Table 2.

**TABLE 2:**

| Radiation-mediated uptake of [3H]-H₂O | | | |
|---|---|---|---|
| SOLVENT | IRRADIATED | NON-IRRADIATED | F(RC)⁺ |
| | Uptake⁺⁺ of [3H] (nmoles/well) | | |
| DMSO | 350 | 1.3 | 269 |
| Formamide | 378 | 3.7 | 102 |
| H₂O | 293 | 1.6 | 183 |

| | | | |
|---|---|---|---|
| ⁺F(RC): Factor of radioconjugation = (irradiated/non-irradiated) uptake of [3H]-H₂O | | | |
| ⁺⁺at saturation | | | |

Example 3: Coupling of proteins to reactive RDPs Conjugands listed in Table 3 (10 mg/ml solutions in 1 mM NaOH) were added to microtiter wells and processed as described in Example 1; ¹²⁵I RNase or ¹²⁵I hemoglobin were diluted with 1 mg/ml unlabeled proteins in borate buffer (0.1 M, pH 8.5), 100 µl/well were added to microtiter plates and kept for sixteen hours at 4°C.

The non-bound proteins were removed as described in Example 2 except that washing solutions were supplemented with 0.05% Tween 20 and PBS was used instead of KCl/glycine solution.

The uptake of proteins were calculated from the total counts added to the wells. The results are shown in Table 3.

**TABLE 3:**

| Coupling of proteins to reactive RDPs | | | | |
|---|---|---|---|---|
| CONJUGAND | NON-IRRADIATED | | IRRADIATED | |
| | RNase | Hemoglobin | RNase | Hemoglobin |
| | Uptake of proteins % | | | |
| None | 0.0 | 0.1 | 0.3 | 0.1 |
| 7-aminoheptanol-1 | 0.0 | 0.0 | 0.0 | 0.0 |
| tryptamine | 0.1 | 0.1 | 0.5 | 0.2 |
| AHE | ND | ND | 0.5 | 0.1 |
| homarylamine | 0.1 | 0.1 | 0.4 | 0.1 |
| o-amino benzoic acid | 0.0 | 0.0 | 0.1 | 0.1 |
| p-aminophenol | 0.5 | 0.1 | 3.7 | 21.3 |
| 2-amino-4-Cl-phenol | 1.5 | 0.1 | 1.2 | 59.9 |
| 5-I-2-amino pyrimidine* | 0.0 | 0.0 | 4.4 | 0.8 |
| o-phenylene diamine | 0.6 | 0.2 | 1.5 | 0.4 |
| m-phenylene diamine | 1.4 | 0.2 | 3.7 | 1.6 |
| m-toluylene diamine | 0.8 | 0.2 | 2.1 | 0.6 |
| 2,6-diamino pyridine | 0.7 | 0.1 | 2.9 | 0.2 |
| 3-O-azo-diamino pyridine | 0.0 | 0.0 | 1.7 | 0.1 |
| 1-naphthylamine* | 0.1 | 0.1 | 1.1 | 0.9 |
| 2-naphthylamine | 0.1 | 0.1 | 0.5 | 0.2 |
| 1,2-naphthylene diamine* | 1.4 | 0.2 | 4.6 | 0.7 |
| 1,8-naphthylene diamine | 2.0 | 0.3 | 4.9 | 0.4 |

| | | | | |
|---|---|---|---|---|
| AHE=DL-2-amino-1(4-Hydroxyphenyl)-ethanol-1 | | | | |
| *=optical quality of PS was impaired during radio-derivatization | | | | |
| ND=not determined The numbers were rounded up to the next tenth digit with .05 cut-off limit. | | | | |

Example 4: EDC-mediated uptake of carboxylic acids by RDPs The uptake of the following carboxylic acids was measured:
A) acetic acid; B) glycine; C) DNP-glycine; D) benzoic acid; E) salicylic acid. Figure 2 shows the effects of radio-derivatization of polystyrene on the EDC-mediated uptake of these carboxylic acids. The irradiation was carried out in the presence of the following solutions (the numbers correspond to the numbering of the x-axis in Figure 2):
1) none;
2) H₂O;
3) NH₃, 25%;
4) ammonium sulphate, saturated;
5) ethylamine-HCl, 90%;
6) 1,3-diamino-2-hydroxy propane 99% (undiluted);
7) 1,4-diaminobutanone.2HCl 20% 1mM NaOH;
8) ethylenediamine.HCl 7.5% in 1 mM NaOH;
9) isobutylamine.HCl 4% in 1 mM NaOH;
10) DL-2-amino-1(4-hydroxyphenyl)-ethan-1-Ol 4% in 1 mM NaOH;
11) 1,6-diamino hexan 14% in 1 mM NaOH;
12) p-aminophenol 4% in 1 mM NaOH;
13) 3,4-diamino pyridine 3.3% in 1 mM NaOH;
14) L-alanine benzyl ester 3.3% in 1 mM NaOH;
15) L-serine benzyl ester 4.4% in 1 mM NaOH;
16) galactosamine 11% in H₂O.

The results are shown in Figure 2. In Chart (a) shaded histograms show uptake of radioactivity without EDC while non-shaded histograms show uptake of radioactivity with EDC. Lower Chart (b) shows the relative uptake: ratio of uptake obtained with irradiated plates versus non-irradiated plates in the presence of EDC. To summarize, the results in Figure 2 show that polystyrene microtiter plates irradiated in the presence of aromatic amines such as p-aminophenol, AHE, L-alanine and benzyl ester become reactive with acetic acid, DNP-Gly or benzoic acid in EDC-mediated reactions. No significant EDC-mediated fixation was observed with glycine (B), most probably because of peptide bond formation as the favored reaction. Low coupling densities were obtained with salicylic acid (E) except with L-alanine benzyl ester-conjugated plates. The selfreactivity of the phenolic hydroxyl in salicylic acid with the mixed EDC-carboxyanhydride derivative may explain the failure of EDC-mediated coupling of this substance to polystyrene.

Example 5: Coupling of proteins to diazotized RDPs Radio-derivatizations were carried out with conjugands listed in Table 4 as described in Example 2. They were diazotized according to the following procedure: Radio-derivatized (and control) plates were precooled in ice-water for 30 minutes. Ice cold NaNO₂ solution (5 mg/ml in 2N HCl) was added and kept in ice-water for 1 hour. The liquid was removed and the plate was washed three times with ice cold water. 100 µl protein solution [1 mg/ml (+ 0.1 µCi trace-labeled RNase and hemoglobin protein) in borate buffer (0.1 M, pH 8.5)] was added to the wells and the coupling was allowed to take place overnight at 4°C; non-bound protein was removed; the plates were washed as described in Example 3 and the uptake of proteins was determined as described in Example 2. The results are shown in Table 4.

**TABLE 4:**

| Coupling of proteins to diazotized radioconjugates | | | | |
|---|---|---|---|---|
| CONJUGAND | NON-IRRADIATED | | IRRADIATED | |
| | RNase | Hemoglobin | RNase | Hemoglobin |
| | Uptake of proteins % | | | |
| None | 0.0 | 0.1 | 0.2 | 0.1 |
| 7-aminoheptanol-1 | 0.0 | 0.0 | 0.0 | 0.0 |
| tryptamine | 0.3 | 0.2 | 0.8 | 0.2 |
| AHE | ND | ND | 0.7 | 0.1 |
| Homarylamine | 0.1 | 0.2 | 0.3 | 0.2 |
| o-amino benzoic acid | 0.0 | 0.0 | 0.1 | 0.1 |
| 5-I-2-amino pyrimidine* | 0.1 | 0.1 | 21.8 | 1.5 |
| o-phenylene diamine | 1.5 | 0.2 | 3.1 | 0.7 |
| m-phenylene diamine | 2.4 | 0.3 | 8.4 | 1.8 |
| m-toluylene diamine | 0.8 | 0.2 | 1.9 | 0.3 |
| 2,6-diamino pyridine | 1.7 | 0.1 | 4.9 | 0.3 |
| 3-O-azo-diamino pyridine* | 0.1 | 0.1 | 5.0 | 0.3 |
| 1-naphthylamine* | 0.4 | 0.2 | 40.8 | 6.2 |
| 2-naphthylene diamine* | 0.2 | 0.1 | 1.3 | 0.2 |
| 1,2-naphthylene diamine | 1.8 | 0.2 | 5.2 | 0.7 |
| 1,5-naphthylene diamine | 2.6 | 0.2 | 9.6 | 0.6 |

| | | | | |
|---|---|---|---|---|
| AHE = DL-2-amino-1 (4-hydroxyphenyl)-ethanol-1 | | | | |
| * Optical quality of PS was impaired during radio-derivatization | | | | |
| ND = not determined The numbers were rounded up to the next tenth digit with 0.05 cut-off limit | | | | |

Example 6: Coupling of proteins to p-aminophenol-polystyrene RDP with EDC and bifunctional reagents P-aminophenol-polystyrene RDP was prepared and processed as described in Example 2. Bifunctional reagents listed in Table 5, namely, ethyldiethylaminopropyl-carbodiimide (EDC); 1,4-butandiol diglycidyl ether (BDE); toluylen-2,4-diisocyanate (TDI) were added to microtiter wells (100 µl/well, 25 mg/ml in K-phosphate buffer (0.2 M, pH 7.2). The plates were kept at room temperature for one hour. The coupling solutions were shaken out and residue removed by impacting on adsorbing paper over a hard surface. Trace-labeled proteins (100 µl/well) were added immediately and kept at 4°C. for sixteen hours and uptake of proteins were measured as described in Example 3. For EDC-mediated coupling 50 µl trace-labeled proteins were added to the wells followed by 50 µl EDC solution (10 mg/ml in 0.1 M NaCl, pH 5); the plates were sealed with adherent tape, kept at room temperature for four hours and uptake of proteins was determined as described above. The results are shown in Table 5.

**TABLE 5:**

| Coupling of proteins to p-aminophenol-PS RDP | | | | | |
|---|---|---|---|---|---|
| PLATE TREATMENT | COUPLING REAGENT | MYOSIN | HEMOGLOBIN | PEPSIN | RNase |
| | | | Uptake of Proteins % | | |
| p-animo-phenol-derivatized | 0 | 2.0 | 21.3 | 0.8 | 3.7 |
| | EDC | 2.5 | 60.1 | 33.2 | 0.0 |
| | glutarald. | 3.0 | 2.0 | 0.3 | 0.2 |
| | epichl.hdr. | 3.1 | 3.1 | 0.0 | 0.0 |
| | BDE | 2.3 | 1.0 | 0.2 | 0.3 |
| | TDI | 8.7 | 17.1 | 9.3 | 10.6 |
| non-irradiated control | 0 | 0.0 | 0.0 | 0.0 | 0.0 |
| | EDC | 0.0 | 0.5 | 0.0 | 0.9 |
| | glutarald. | 0.0 | 0.2 | 0.0 | 0.1 |
| | epichl.hdr. | 0.4 | 0.0 | 0.0 | 0.0 |
| | BDE | 0.0 | 0.0 | 0.0 | 0.0 |
| | TDI | 1.1 | 1.0 | 1.5 | 1.0 |
| The numbers were rounded up to the next digit with 0.05 cut-off limit. | | | | | |

Example 7: EDC-mediated coupling of Penicillin G to water-polystyrene RDP
Polystyrene microtiter plates were irradiated in a water-containing atmosphere as described in Example 1. Penicillin G (2 mM unlabeled + 0.1 µCi [14C] penicillin G in 50 µl 0.1 M NaCl, pH 3) and the same volume of EDC (1 mg/ml in 0.1 M NaCl, pH 3) was added and kept at room temperature for two hours. The non-bound penicillin G was removed and the plates were processed as described in Example 1. Coupling densities in the range of 50-100 pmole penicillin G/cm² were obtained.

Example 8: EDC-mediated coupling of DNP-aminoacids to water-polystyrene RDP
Microtiter plates were radio-derivatized as described in the previous example. DNP-glycine (1 mM + 0.1 µCI DNA[3H]glycine in 50 µl 0.1 M NaCl, pH 3) and the same volume of EDC (1 mg/ml in 0.1 M NaCl, pH 3) were incubated in the microtiter plate wells at room temperature for at least one hour. The non-bound DNP-glycine was removed and the plates were processed as described in Example 1. Coupling densities ranged between 100-150 pmoles/cm².

Example 9: Coupling of myosin to water-polystyrene RDP with oxyranes
Microtiter plates were activated as described in the previous example. They were then incubated with BDE (25 mg/ml, in 100 µl phosphate buffer, pH 7.2) for one hour. The coupling solution was removed; myosin (1 mg/ml + 0.1 µCi ¹²⁵I myosin in 100 µl phosphate buffer pH 7.2) was added and coupling was allowed to take place for fourteen to sixteen hours at 4°C. The non-bound myosin was removed and the plates were processed as described in Example 3. The coupling densities ranged between 20-30 pmol myosin/cm².

Example 10: Coupling of myosin to water-polystyrene RDP with TDC
The same method was used as described in the previous Example, but TDC was used instead of BDE. Coupling densities in the range of 15-20 pmoles myosin/cm² were obtained.

Example 11: Coupling of RNase to water-polystyrene RDP with TDC
The same method was used as described in Example 10, but RNAse [+¹²⁵I-RNAse] was used instead of myosin. The obtainable coupling densities ranged between 2-2.5 nmoles of RNase/cm².

Example 12: Coupling of hemoglobin to p-aminophenylpolystyrene RDP
Para-aminophenyl-polystyrene RDP was obtained as described in Example 4, and hemoglobin (1 mg/ml + 0.1 µCi ¹²⁵I hemoglobin in 100 pl 0.1 M NaCl, pH 3) was added and kept at room temperature for four hours. The non-bound hemoglobin was removed and the plate was processed as described in Example 3. Approximately 1 nmole hemoglobin/cm² coupling densities were obtained.

Example 13: EDC-mediated coupling of pepsin to p-aminophenyl polystyrene RDP
Para-aminophenyl-polystyrene RDP was obtained as described in Example 4 and pepsin (1 mg/ml + 0.1 µCi ¹²⁵I pepsin in 100 µl 0.1 M NaCl, pH5) was added, followed by the addition of the same volume of EDC (10 mg/ml in the same solution). Following four hours of incubation at room temperature the non-bound pepsin was removed and the plate was processed as described in Example 12. Approximately 1 nmole pepsin/cm² coupling densities were obtained.

Example 14: EDC-mediated coupling of penicillin G to para-aminophenyl-polystyrene RDP
Para-aminophenyl-polystyrene RDP was obtained as described in Example 4, penicillin G (2 mg/ml + 0.1 µCi 14C penicillin G in 100 µl 0.1 M NaCl, pH 5) plus the same volume of EDC (10 mg/ml in 0.1 M NaCl, pH 5) was added and kept at room temperature for four hours. The non-bound penicillin was removed and the plate was processed as described in Example 1. Approximately 3 nmoles/cm² coupling densities were obtained.

Example 15: Coupling of RNAse to para-aminophenyl-polystyrene RDP
Para-aminophenyl-polystyrene RDP was obtained as described in Example 4; TDC was used as coupling reagent as described in Example 10; RNAse (1 mg/ml + 0.1 µCi ¹²⁵I RNAse in 100 µl 0.1 M borate buffer, pH 8.5) was added and kept at 4°C for fourteen-sixteen hours. The non-bound RNAse was removed and the plate was washed and treated as described in Example 12. Approximately 1 nmole RNAse/cm² coupling densities were obtained.

Example 16: EDC-mediated coupling of acetic acid to AHE-polystyrol RDP
AHE-polystyrol RDP was obtained as described in Example 4 and acetic acid (0.1 M + 0.1 µCi [3H] acetic acid in 100 pl 0.1 M NaCl, pH 5) plus the same volume of EDC (20 mg/ml in the same solution) was added and kept at room temperature for four hours. The non-bound acetic acid was removed and the plate was processed as described in Example 1. Approximately 3 nmoles acetate/cm² coupling densities were obtained.

Example 17: EDC-mediated coupling of penicillin G to AHE-polystyrene RDP
The same method as described in the previous example was used but penicillin G (20 mM) was used instead of acetic acid. Approximately 1.5 nmoles of penicilling G/cm² coupling densities were obtained.

Example 18: EDC-mediated coupling of benzoic acid to AHE-polystyrene RDP
The procedure described in Example 16 was followed but benzoic acid (10 mM) was used instead of acetic acid. The coupling densities ranged between 0.6-1 nmoles benzoic acid/cm².

Example 19: EDC-mediated coupling of prostaglandin El to AHE-polystyrene RDP
The same procedure as described in Example 16 was followed, but prostaglandin El (1 mM) was used instead of acetic acid. The range of coupling densities were 0.5-1 nmoles prostaglandin E1/cm².

Example 20: EDC-mediated coupling of DNP-aminoacids to AHE-polystyrene RDP
The same procedure was followed as described in Example 16, but DNP-glycine (10 mM + 0.1 µCi DNP[3H]gly) was used. Approximately 5 nmoles DNP-glycine/cm² coupling densities were obtained.

Example 21: EDC-mediated coupling of pepsin to AHE-polystyrene RDP
The same procedure was followed for the preparation of AHE-PS RDP as described in Example 16, but pepsin (1 mg/ml + 0.1 µCi ¹²⁵I pepsin) was used instead of acetic acid. The plates were processed as described in Example 9. The coupling densities ranged between 0.8-1 nmoles pepsin/cm².

Example 22: Binding assay for DNP-specific IgE antibodies Microtiter plates containing covalently bound DNP-glycine, obtained by methods described in Examples 4, 8 and 20 were used. DNP-binding mouse monoclonal IgE (trace-labeled with ¹²⁵I) was added and incubated at 4°C. for sixteen hours. The non-bound IgE was removed and the residual radioactivity (ranging between 30-40% of total IgE added) was determined. The intra- and inter-assay CV were under 3%. The same binding assay has been used to screen large numbers of drugs for multispecific interactions with IgE in studies on drug allergies., (Varga, J.M., Klein, G. and Fritsch, P.: J. Invest. Dermatol., Vol. 92, 1989, pp. 537).

## Claims

1. A method for preparing a radio-derivatized polymer comprising contacting a radiolysable polymer and a nonpolymerizable conjugand in liquid form, wherein at least one of said radiolysable polymer and said conjugand is irradiated, with high energy radiation, selected from gamma rays with an irradiation dose in the range of 1 to 15 Mrads and an irradiation intensity in the range of 0,1 to 0,2 Mrads/h, and electron-radiation that is capable of radiolysing the polymer and/or conjugand, thereby linking said conjugand to said polymer by means of said irradiation.

2. The method of claim 1, wherein said radiolysable polymer is irradiated.

3. The method of claim 2, wherein said radiolysable polymer is irradiated prior to said contacting step.

4. The method of claim 1, wherein said conjugand is irradiated.

5. The method of claim 4, wherein said conjugand is irradiated prior to said contacting step.

6. The method of claim 1 wherein both said polymer and said conjugand are irradiated.

7. The method of claim 6 wherein said radiolysable polymer and said conjugand are irradiated prior to said contacting step.

8. The method of claim 6 wherein said radiolysable polymer and said conjugand are irradiated after said contacting step.

9. The method of claim 1, wherein said conjugand is selected from the group consisting of quinones, compounds from which quinones are generated under irradiation, aromatic compounds, nucleophile-containing substances, amino group-containing compounds, aromatic amine compounds, carbohydrates and derivatives thereof, carboxyl-containing compounds, protein-modifying reagents and water.

10. The method of claim 1, wherein said radiolysable polymer is polystyrene.

11. The method of claim 1, which further comprises linking said radio-derivatized polymer to a ligand selected from the group consisting of proteins, spacers, chelators, ions, cofactors, coenzymes, hormones, drugs, dyes, lipids, carbohydrates, lectins, nucleic acids, enzymes, haptens, antigens, allergens, antibodies, viruses, microorganisms, eukaryotic cells and representative components thereof.

12. The method of claim 1 wherein the conjugand is water and the radio-derivatized product is reactive with at least one carboxyl-containing compound in the presence of at least one activator.

13. The method of claim 1 wherein the conjugand is an amino group-containing compound and the radio-derivatized product is reactive with carboxyl-containing compounds in the presence of at least one activator.

14. The method of claim 1 wherein the conjugand is a nucleophile-containing substance and the product is a nucleophile-functionalized radio-derivatized polymer.

15. The method of claims 12, 13 or 14, wherein the radio-derivatized polymer is reactive with bi-functional reagents.

16. The method of claim 1 wherein the conjugand is an aromatic amine and the radio-derivatized polymer id diazotized.

17. The method of claim 1, wherein the conjugand is a carbohydrate or a derivative thereof thereby forming a glycophase radio-derivatized polymer wherein said glycophase radio-derivatized polymer is further activated.

18. The method of claim 17, wherein said radio-derivatized glycophase polymer is further activated with CNBr or periodate to react with nucleophiles.

19. The method of claim 1, wherein the conjugand is a carboxyl-containing compound and the resulting radio-derivatized polymer is further activated to react with nucleophiles.

20. The method of claim 18, wherein said carboxylated radio-derivatized product is further activated with carbodiimides to react with nucleophiles.

21. The method of claim 1, wherein the conjugand is a protein-modifying reagent and the radio-derivatized polymer is a nucleophile-reactive polymer.

22. A radio-derivatized polymer formed by contacting a radiolysable polymer and a nonpolymerizable conjugand, wherein at least one of said radiolysable polymer and said conjugand is irradiated, thereby, linking said radiolysable polymer to said conjugand by means of said irradiation wherein said conjugand is selected from the group consisting of quinones, compounds from which quinones are generated under irradiation, aromatic compounds, nucleophile-containing compounds, amino group-containing compounds, aromatic amine compounds, carbohydrates and derivatives thereof, carboxyl-containing compounds, protein-modifying reagents and water, with the proviso that said conjugand is capable of linking with said polymer only under high energy radiation.

23. A radio-derivatized polymer according to claim 21, wherein said linkage is formed by irradiating said radiolysable polymer prior to said contacting step with said conjugand.

24. A radio-derivatized polymer according to claim 21, wherein said linkage is formed by irradiating said conjugand prior to said contacting step with said radiolysable polymer.

25. A radio-derivatized polymer according to claim 21 wherein said linkage is formed by irradiating said radiolysable polymer and said conjugand prior to said contacting step.

26. A radio-derivatized polymer according to claim 21 wherein said linkage is formed by irradiating said radiolysable polymer and said conjugand after said contacting step.

27. A radio-derivatized polymer according to claim 21, wherein said radio-derivatized polymer is further linked to a ligand.

28. A radio-derivatized polymer according to claim 26, wherein said ligand is selected from the group consisting of proteins, spacers, chelators, ions, cofactors, coenzymers, hormones, drugs, dyes, lipids, carbohydrates, lectins, nucleic acids, enzymes, haptens, antigens, allergens, antibodies, viruses, microoragnisms, eukaryiotic cells and representative components thereof.

29. A radio-derivatized polymer according to claim 21, wherein said polymer is polystyrene.

## Patentansprüche

1. Verfahren zur Herstellung eines radioderivatisierten Polymers, welches das In-Kontakt-Bringen eines radiolysierbaren Polymers und eines nichtpolymerisierbaren Konjuganden in flüssiger Form umfaßt, wobei mindestens einer von dem radiolysierbaren Polymer und dem Konjuganden mit Hochenergiestrahlung bestrahlt wird, die aus Gammastrahlen mit einer Bestrahlungsdosis im Bereich von 1 bis 15 Mrad und einer Bestrahlungsstärke im Bereich von 0,1 bis 0,2 Mrad/h und aus Elektronenstrahlung, die zur Radiolyse des Polymers und/oder Konjuganden imstande ist, ausgewählt wird, wodurch der Konjugand mit dem Polymer durch die Bestrahlung verbunden wird.

2. Verfahren nach Anspruch 1, wobei das radiolysierbare Polymer bestrahlt wird.

3. Verfahren nach Anspruch 2, wobei das radiolysierbare Polymer vor dem Kontaktierschritt bestrahlt wird.

4. Verfahren nach Anspruch 1, wobei der Konjugand bestrahlt wird.

5. Verfahren nach Anspruch 4, wobei der Konjugand vor dem Kontaktierschritt bestrahlt wird.

6. Verfahren nach Anspruch 1, wobei das radiolysierbare Polymer und der Konjugand bestrahlt werden.

7. Verfahren nach Anspruch 6, wobei das radiolysierbare Polymer und der Konjugand vor dem Kontaktierschritt bestrahlt werden.

8. Verfahren nach Anspruch 6, wobei das radiolysierbare Polymer und der Konjugand nach dem Kontaktierschritt bestrahlt werden.

9. Verfahren nach Anspruch 1, wobei der Konjugand ausgewählt wird aus der Gruppe bestehend aus Chinonen, Verbindungen, aus welchen Chinone unter Bestrahlung erzeugt werden, aromatischen Verbindungen, nukleophilhältigen Substanzen, Aminogruppen-hältigen Verbindungen, aromatischen Aminverbindungen, Kohlenhydraten und Derivaten davon, carboxylhältigen Verbindungen, proteinmodifizierenden Reagentien und Wasser.

10. Verfahren nach Anspruch 1, wobei das radiolysierbare Polymer Polystyrol ist.

11. Verfahren nach Anspruch 1, das ferner das Verbinden des radioderivatisierten Polymers mit einem Liganden umfaßt, der ausgewählt wird aus der Gruppe bestehend aus Proteinen, Spacern, Chelatbildnern, Ionen, Kofaktoren, Koenzymen, Hormonen, Arzneidrogen, Farbstoffen, Lipiden, Kohlenhydraten, Lektinen, Nukleinsäuren, Enzymen, Haptenen, Antigenen, Allergenen, Antikörpern, Viren, Mikroorganismen, eukaryotischen Zellen und repräsentativen Komponenten davon.

12. Verfahren nach Anspruch 1, wobei der Konjugand Wasser ist und das radioderivatisierte Produkt mit mindestens einer carboxylhältigen Verbindung in Gegenwart von mindestens einem Aktivator reaktionsfähig ist.

13. Verfahren nach Anspruch 1, wobei der Konjugand eine Aminogruppen-hältige Verbindung ist und das radioderivatisierte Produkt mit carboxylhältigen Verbindungen in Gegenwart von mindestens einem Aktivator reaktionsfähig ist.

14. Verfahren nach Anspruch 1, wobei der Konjugand eine nukleophilhältige Substanz ist und das Produkt ein nukleophilfunktionalisiertes radioderivatisiertes Polymer ist.

15. Verfahren nach Anspruch 12, 13 oder 14, wobei das radioderivatisierte Polymer mit bifunktionellen Reagentien reaktionsfähig ist.

16. Verfahren nach Anspruch 1, wobei der Konjugand ein aromatisches Amin ist und das radioderivatisierte Polymer diazotiert ist.

17. Verfahren nach Anspruch 1, wobei der Konjugand ein Kohlenhydrat oder ein Derivat davon ist, wodurch ein Glykophasen-radioderivatisiertes Polymer gebildet wird, wobei das Glykophasen-radioderivatisierte Polymer weiter aktiviert wird.

18. Verfahren nach Anspruch 17, wobei das Glykophasen-radioderivatisierte Polymer weiter mit CNBr oder Periodat aktiviert ist, um mit Nukleophilen zu reagieren.

19. Verfahren nach Anspruch 1, wobei der Konjugand eine carboxylhältige Verbindung ist und das erhaltene radioderivatisierte Polymer weiter aktiviert wird, um mit Nukleophilen zu reagieren.

20. Verfahren nach Anspruch 18, wobei das carboxylierte radioderivatisierte Produkt weiter mit Carbodiimiden aktiviert wird, um mit Nukleophilen zu reagieren.

21. Verfahren nach Anspruch 1, wobei der Konjugand ein proteinmodifizierendes Reagens ist und das radioderivatisierte Polymer ein nukleophil-reaktionsfähiges Polymer ist.

22. Radioderivatisiertes Polymer, das durch das In-Kontakt-Bringen eines radiolysierbaren Polymers und eines nichtpolymerisierbaren Konjuganden gebildet wird, wobei mindestens einer von dem radiolysierbaren Polymer und dem Konjuganden bestrahlt wird, wodurch das radiolysierbare Polymer mit dem Konjuganden durch die Bestrahlung verbunden wird, wobei der Konjugand ausgewählt wird aus der Gruppe bestehend aus Chinonen, Verbindungen, aus welchen Chinone unter Bestrahlung erzeugt werden, aromatischen Verbindungen, nukleophilhältigen Substanzen, Aminogruppen-hältigen Verbindungen, aromatischen Aminverbindungen, Kohlenhydraten und Derivaten davon, carboxylhältigen Verbindungen, proteinmodifizierenden Reagentien und Wasser, unter der Voraussetzung, daß der Konjugand imstande ist, sich mit dem Polymer nur unter Hochenergiebestrahlung zu verbinden.

23. Radioderivatisiertes Polymer nach Anspruch 21, wobei die Verbindung durch Bestrahlung des radiolysierbaren Polymers vor dem Kontaktierschritt mit dem Konjuganden gebildet ist.

24. Radioderivatisiertes Polymer nach Anspruch 21, wobei die Verbindung durch Bestrahlung des Konjuganden vor dem Kontaktierschritt mit dem radiolysierbaren Polymer gebildet ist.

25. Radioderivatisiertes Polymer nach Anspruch 21, wobei die Verbindung durch Bestrahlung des radiolysierbaren Polymers und des Konjuganden vor dem Kontaktierschritt gebildet ist.

26. Radioderivatisiertes Polymer nach Anspruch 21, wobei die Verbindung durch Bestrahlung des radiolysierbaren Polymers und des Konjuganden nach dem Kontaktierschritt gebildet ist.

27. Radioderivatisiertes Polymer nach Anspruch 21, wobei das radiolysierbare Polymer zusätzlich mit einem Liganden verbunden ist.

28. Radioderivatisiertes Polymer nach Anspruch 26, wobei der Ligand ausgewählt wird aus der Gruppe bestehend aus Proteinen, Spacern, Chelatbildnern, Ionen, Kofaktoren, Koenzymen, Hormonen, Arzneidrogen, Farbstoffen, Lipiden, Kohlenhydraten, Lektinen, Nukleinsäuren, Enzymen, Haptenen, Antigenen, Allergenen, Antikörpern, Viren, Mikroorganismen, eukaryotischen Zellen und repräsentativen Komponenten davon.

29. Radioderivatisiertes Polymer nach Anspruch 21, wobei das Polymer Polystyrol ist.

## Revendications

1. Procédé de préparation d'un polymère dérivé d'un radio-élément, comprenant la mise en contact d'un polymère radiolisable et d'un conjuguant non polymérisable se présentant sous forme liquide, dans lequel au moins l'un parmi ledit polymère radiolisable et ledit conjuguant est irradié avec un rayonnement à haute énergie, sélectionné parmi des rayons gamma avec une dose d'irradiation comprise dans la plage allant de 1 à 15 Mrads et une intensité de rayonnement comprise dans la plage allant de 0,1 à 0,2 Mrads par heure, et un rayonnement d'électrons qui peut radioliser le polymère et/ou le conjuguant liant de ce fait ledit conjuguant audit polymère au moyen dudit rayonnement.

2. Procédé selon la revendication 1, dans lequel ledit polymère radiolisable est irradié.

3. Procédé selon la revendication 2, dans lequel ledit polymère radiolisable est irradié avant ladite étape de mise en contact.

4. Procédé selon la revendication 1, dans lequel ledit conjuguant est irradié.

5. Procédé selon la revendication 4, dans lequel ledit conjuguant est irradié avant ladite étape de mise en contact.

6. Procédé selon la revendication 1, dans lequel à la fois ledit polymère et ledit conjuguant sont irradiés.

7. Procédé selon la revendication 6, dans lequel ledit polymère radiolisable et ledit conjuguant sont irradiés avant ladite étape de mise en contact.

8. Procédé selon la revendication 6, dans lequel ledit polymère radiolisable et ledit conjuguant sont irradiés après ladite étape de mise en contact.

9. Procédé selon la revendication 1, dans lequel ledit conjuguant est sélectionné dans le groupe comprenant les quinones, des composés à partir desquels des quinones sont générés sous irradiation, des composés aromatiques, des substances contenant un nucléophile, des composés contenant un groupe amino, des composés d'amines aromatiques, des carbohydrates et des dérivés de ces derniers, des composés contenant du carboxyde, des réactifs modifiant une protéine et de l'eau.

10. Procédé selon la revendication 1, dans lequel ledit polymère radiolisable est du polystyrène.

11. Procédé selon la revendication 1, comprenant en outre la liaison dudit polymère dérivé d'un radio élément à un liant sélectionné dans le groupe comprenant des protéines, des éléments d'espacement, des agents de chellation, des ions, des adjuvants, des coenzymes, des hormones, des médicaments, des colorants, des lipides, des carbohydrates, des lectines, des acides nucléiques, des enzymes, des haptènes, des antigènes, des allergènes, des anticorps, des virus, des micro-organismes, des cellules eucaryotiques et des composants représentatifs de ces derniers.

12. Procédé selon la revendication 1, dans lequel le conjuguant est de l'eau et le produit dérivé d'un radio-élément réagit avec au moins un composé contenant du carboxyle en la présence d'au moins un activateur.

13. Procédé selon la revendication 1, dans lequel le conjuguant est un composé contenant un groupe amino et le produit dérivé d'un radio élément réagit avec des composés contenant du carboxyle en la présence d'au moins un activateur.

14. Procédé selon la revendication 1, dans lequel le conjuguant est une substance contenant un nucléophile et le produit est un polymère dérivé d'un radio-élément, à fonctionnalité nucléophile.

15. Procédé selon la revendication 12, 13 ou 14, dans lequel le polymère dérivé d'un radio-élément réagit avec des réactifs bifonctionnels.

16. Procédé selon la revendication 1, dans lequel le conjuguant est une amine aromatique et le polymère dérivé d'un radio-élément est diazotisé.

17. Procédé selon la revendication 1, dans lequel le conjuguant est un carbohydrate ou un dérivé de ce dernier, formant de ce fait un polymère dérivé d'un radio-élément à base de glycophase, dans lequel ledit polymère dérivé d'un radio élément à base de glycophase est encore activé.

18. Procédé selon la revendication 17, dans lequel ledit polymère à base de glycophase dérivé d'un radio-élément est en outre activé avec un CNBr ou un périodate afin de réagir avec des nucléophiles.

19. Procédé selon la revendication 1, dans lequel le conjuguant est un composé contenant du carboxyle et le polymère résultant dérivé d'un radio-élément est en outre activé afin de réagir avec des nucléophiles.

20. Procédé selon la revendication 18, dans lequel ledit produit carboxylé dérivé d'un radioélément est en outre activé avec des carbodiimides afin de réagir avec des nucléophiles.

21. Procédé selon la revendication 1, dans lequel le conjuguant est un réactif modifiant une protéine et le polymère dérivé d'un radio-élément est un polymère réagissant avec un nucléophile.

22. Polymère dérivé d'un radio-élément formé par la mise en contact d'un polymère radiolisable et d'un conjuguant non polymérisable, dans lequel au moins l'un parmi ledit polymère radiolisable et ledit conjuguant est irradié, liant de ce fait ledit polymère radiolisable et ledit conjuguant est irradié, liant de ce fait ledit polymère radiolisable audit conjuguant au moyen de ladite irradiation, dans lequel ledit conjuguant est sélectionné dans le groupe comprenant les quinones, des composés à partir desquels les quinones sont générés sous l'irradiation, des composés aromatiques, des composés contenant un nucléophile, des composés contenant un groupe amino, des composés d'amines aromatiques, des carbohydrates et des dérivés de ces derniers, des composés contenant du carboxyle, des réactifs modifiant une protéine et de l'eau, dans la mesure où ledit conjuguant peut assurer une liaison avec ledit polymère seulement sous un rayonnement à haute énergie.

23. Polymère dérivé d'un radio-élément selon la revendication 21, dans lequel ladite liaison est formée par une irradiation dudit polymère radiolisable avant ladite étape de mise en contact avec ledit conjuguant.

24. Polymère dérivé d'un radio-élément selon la revendication 21, dans lequel ladite liaison est formée irradiation dudit conjuguant et avant ladite étape de mise en contact avec ledit polymère radiolisable.

25. Polymère dérivé d'un radio-élément selon la revendication 21, dans lequel ladite liaison est formée par irradiation dudit polymère radiolisable et dudit conjuguant avant ladite étape de mise en contact.

26. Polymère dérivé d'un radio-élément selon la revendication 21, dans lequel ladite liaison est formée par irradiation dudit polymère radiolisable et dudit conjuguant après ladite étape de mise en contact.

27. Polymère dérivé d'un radio-élément selon la revendication 21, dans lequel ledit polymère dérivé d'un radio-élément est en outre relié à un liant.

28. Polymère dérivé d'un radio-élément selon la revendication 26, dans lequel ledit liant est sélectionné dans le groupe comprenant des protéines, des éléments d'espacement, des agents de chellation, des ions, des adjuvants, des coenzymes, des hormones, des médicaments, des colorants, des lipides, des carbohydrates, des lectines, des acides nucléiques, des enzymes, des aptènes, des antigènes, des allergènes, des anticorps, des virus, des micro-organismes, des cellules eucaryotiques et des composants représentatifs de ces derniers.

29. Polymère dérivé d'un radio-élément selon la revendication 21, dans lequel ledit polymère est du polystyrène.
